# EUROPEAN PATENT APPLICATION

(11) **EP 1 674 484 A2**
(43) Date of publication of application: **28.06.2006**
(21) Application number: 06075266.4
(22) Date of filing: 17.12.1997
(51) Int. Cl.: C08F 8/00, C07C 45/00, C07C 45/45, C07C 45/51, C07C 209/00, C07C 209/42, C07C 239/08, C07C 259/08, C07D 307/32, C07D 311/68, C07D 311/82, C07D 313/12

(54) **Process for the solid phase synthesis of aldehydes, ketones, oximes, amines and hydroxamic acid componds**

(30) Priority: 19.12.1996 US 32453 P; 24.12.1996 US 33881 P
(62) Divisional of application: 97953458.3
(71) Applicant: Aventis Pharmaceuticals Products Inc., Bridgewater, NJ 08807-0800 (US)
(72) Inventor: Salvino, Joseph M., Schenksville, PA 19473 (US); Morton, George C., Collegeville, PA 19426 (US); Mason, Helen J., Skillman, NJ 08558 (US); Labaudiniere, Richard F., Sherborn, MA 01770 (US)
(74) Representative: Jones, Stephen Anthony

(57) **Abstract**

This invention is directed to polymeric hydroxylamine resin compounds and to processes for the solid-phase synthesis of aldehydes, ketones, oximes, amines and hydroxamic acid compounds useful thereof.

## Description

### FIELD OF THE INVENTION

This invention is directed processes for the solid-phase synthesis of aldehydes, ketones, oximes, amines and hydroxamic acid compounds and to polymeric hydroxylamine resin compounds useful therefor.

### BACKGROUND OF THE INVENTION

Solid-phase synthetic techniques, in which a reagent is immobilized on a polymeric material which is inert to the reagents and reaction conditions employed, as well as being insoluble in the media used, are important tools for preparing amides, peptides and hydroxamic acids. For solid phase peptide synthesis, a summary of the many techniques may be found in J.M. Stewart and J.D. Young, *Solid Phase Peptide Synthesis,* 2nd. Ed., Pierce Chemical Co. (Chicago, IL, 1984); J. Meienhofer, *Hormonal Proteins and Peptides,* vol. 2, p. 46, Academic Press (New York), 1973; and E. Atherton and R.C. Sheppard, *Solid Phase Peptide Synthesis: A Practical Approach,* IRL Press at Oxford University Press (Oxford, 1989). For the use of solid phase methodology in the preparation of non-peptide molecules see Leznoff, C.C., *Acc. Chem. Res.,*11*,* 327-333 (1978).

A number of polymeric reagents have found synthetic use in simple functional group transformations. *See* A. Akelah and D.C. Sherrington, Application of Functionalized Polymers in Organic Synthesis, *Chem Rev.,* 81, 557-587 (1981) and W. T. Ford and E. C. Blossey, *Polymer Supported Reagents, Polymer supported Catalysts, and Polymer Supported Coupling Reactions, in Preparative Chemistry using Supported Reagents,* Pierre Laszlo, ed., Academic Press, Inc, 193-212 (1987). For the use of polymeric reagents in oxidation reactions see J. M. J. Frechet et al., J. *Org. Chem.,* 43, 2618 (1978) and G. Cainelli et al., J. *Am. Chem. Soc., 98,* 6737 (1976). For the use of polymeric reagents in halogenation reactions see J. M. J. Frechet et al., *J. Macromol. Sci. Chem.,* A-11, 507 (1977) and D. C. Sherrington et al., *Eur. Polym. J.,* 13, 73, (1977). For the use of polymeric reagents in epoxidation reactions see J. M. J. Frechet et al., *Macromolecules,* 8,130 (1975) and C. R. Harrison et al., J. *Chem. Soc. Chem. Commun.,* 1009 (1974). For the use of polymeric reagents in acylation reactions see M. B. Shambhu et al., *Tet. Lett.,* 1627 (1973) and M. B. Shambhu et al., J. *Chem. Soc. Chem. Commun.,* 619 (1974). For the use of polymeric reagents in Wittig reactions see S. V. McKinley et al., *J. Chem. Soc. Chem. Commun.*, 134 (1972).

Polymeric reagents have also found widespread use in combinatorial synthesis and for preparing combinatorial libraries. *See* F. Balkenhohl et al., *Angew. Chem. Int. Ed. Engl.,* 35, 2288-2337 (1996) and L.A. Thompson et al., *Chem Rev.,* 96, 555-600 (1996).

A polymeric reagent has the advantage of ease of separation from low molecular weight reactants or products by filtration or selective precipitation. The polymeric reagent can also be used in excess to effect fast and quantitative reactions such as in the case of acylations, or a large excess of reactants may be used to drive the equilibrium of the reaction towards product formation to provide essentially quantitative conversion to product, as see in solid phase peptide synthesis. A further advantage of supported reagents and catalysts is the fact that they are recyclable and that they lend easily to automated processes. In addition, supported analogs of toxic and odorous reagents are safer to use.

PCT application publication no. W096/26223 discloses the synthesis of hydroxamic acid compounds using a solid phase hydroxylamine substrate.

Prasad et al. disclose a O-methylhydroxylamine-polystyrene resin compound in J. *Steroid Biochem.,*18, 257-261 (1983).

### SUMMARY OF THE INVENTION

This invention is directed to a process for the preparation of a ketone compound of formula 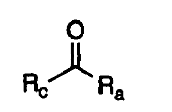 wherein R_{c} and Rₐ are independently aliphatic or aromatic, comprising
(a) reacting an N-alkylated polymeric hydroxamic acid resin compound of formula 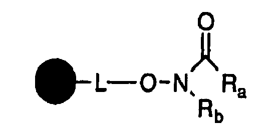 wherein 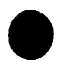 is a solid support, L is absent or a linking group and R_{b} is aliphatic or aryl
   with an organometallic reagent of formula R_{c}M wherein R_{c} is an aliphatic or aryl anion and M is a metal cation; and
(b) liberating the ketone compound from the resin.

In another aspect, this invention is directed to a process for the preparation of an aldehyde compound of formula R₂CHO wherein Rₐ is defined above, comprising
(a) reacting an N-alkylated polymeric hydroxamic acid resin compound of formula 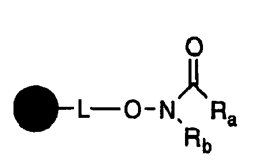 wherein 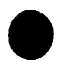 ,L and Rₐ, and R_{b} are defined above; with a reducing agent; and
(b) liberating the aldehyde compound from the resin.

In another aspect, this invention is directed to a process for the preparation of a N-alkylated polymeric hydroxamic acid resin compound of formula 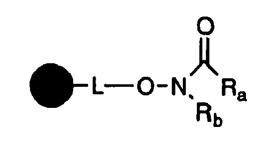 wherein 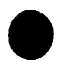 L and Rₐ and R_{b} are defined above, comprising
(a) coupling a carboxylic acid compound of formula RₐCO₂H with a polymeric hydroxylamine resin compound of formula 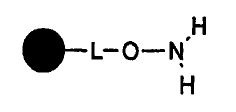 to form a polymeric hydroxamic acid resin compound of formula 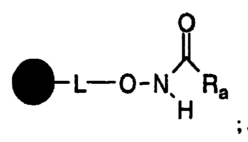 and
(b) reacting the polymeric hydroxamic acid resin compound with an alkylating agent of formula R_{b}LG wherein LG is a leaving group.

In another aspect, this invention is directed to a process for the preparation of a N-alkylated polymeric hydroxamic acid resin compound of formula 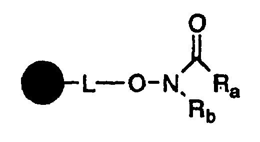 wherein 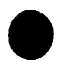 ,L and Rₐ and R_{b} are defined above, comprising
(a) reacting a N-protected polymeric hydroxamic acid resin compound of formula 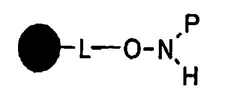 wherein P is an amine protecting group, with an alkylating agent of formula R_{b}LG wherein LG is defined above, to form a polymeric N-protected N-alkylated hydroxylamine resin compound of formula 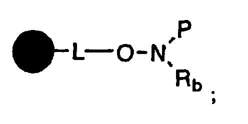
(b) removing the amine protecting group to form a polymeric N-alkylated hydroxylamine resin compound of formula 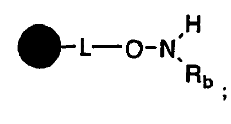 and
(c) coupling the polymeric N-alkylated hydroxylamine resin compound with a carboxylic acid compound of formula RₐCO₂H.

In another aspect, this invention is directed to a process for preparing a hydroxamic acid compound of formula 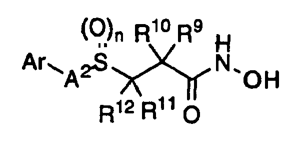 wherein

A² is a direct bond or an alkylene, or NR¹³ wherein R¹³ is hydrogen or alkyl;

R⁹ is a group of formula -L¹-R¹⁴, where L¹ represents a direct bond or a straight- or branched C₁₋₆alkylene chain optionally substituted by alkoxy, aryl, carboxy, cyano, cycloalkyl, halogen, heteroaryl, hydroxyl, or oxo, and R¹⁴ is hydrogen, aryl, carboxy, cyano, cycloalkyl, cycloalkenyl, cyclocarbamoyl, cycloimidyl, heterocycloalkyl, heteroaryl, -NH-C(=O)-NH₂, (N-cazbamoylxyclic amine, -C=N-O-C(=O)-NH₂, -C(=O)-NY¹ Y² [where Y¹ and Y² are independently hydrogen, alkyl, arylalkyl, and aryl, or the substituent Y¹Y²N- forms a 4-6 membered cyclic amine which optionally contains an additional heteroatom selected from O S, NH or NR¹³], -NY¹ SO₂aryl, -NHR¹³, -SR¹³ or -OR¹³; or a group L²-R¹⁵ where L² represents a straight- or branched-carbon chain comprising from 2 to about 6 carbon atoms, optionally substituted with carboxy or cyano, which contains a double or triple carbon-carbon bond, or is interrupted by an oxygen or sulfur atom, a phenylene, imino (-NH-) or alkylimino linkage, or a sulfinyl or sulfonyl group, and R¹ is hydrogen, aryl, carboxy, cyano, cycloalkyl, cycloalkenyl, heterocycloalkyl or heteroaryl; or R⁹ and R¹⁰ taken together with the atom to which they are attached form a ring; or R⁹ and R¹¹ taken together with the atoms through which they are attached form a ring;

R¹⁰ and R¹² are independently hydrogen or alkyl; or R¹⁰ and R¹² together form a bond;

R¹¹ represents a group -L³-R¹⁶ where L³ represents a direct bond or a straight- or branched C₁₋₆alkylene chain optionally substituted by by alkoxy, aryl, carboxy, cyano, cycloallcyl, halogen, heteroaryl, hydroxyl, or oxo; or L³ represents a straight- or branched-carbon chain comprising from 2 to about 6 carbon atoms which contains a double or triple carbon-carbon bond, or is interrupted by an oxygen or sulfur atom, a phenylene, imino (-NH-) or alkylimino linkage, or a sulfinyl or sulfonyl group; and R¹⁶ is hydrogen, aryl, cycloalkyl, cycloalkenyl, cyclocarbamoyl, cycloimidyl, heterocycloalkyl, heteroaryl, -NH-C(=O)-NH₂, (N-carbamoyl)cyclic amine, -C=N-O-C(=O)-NH₂, -C(=O)-NY¹Y² [where Y¹ and Y² are independently hydrogen, alkyl, arylalkyl, and aryl, or the substituent Y¹ Y²N- forms a 4-6 membered cyclic amine which optionally contains an additional heteroatom selected from O, S, NH or NR¹³], -NY¹ SO₂aryl, or R¹¹ and R⁹ together with the atoms to which they are attached form a ring; or R¹¹ and R¹² together with the atom to which they are attached form a ring;

Ar is a group chosen from: 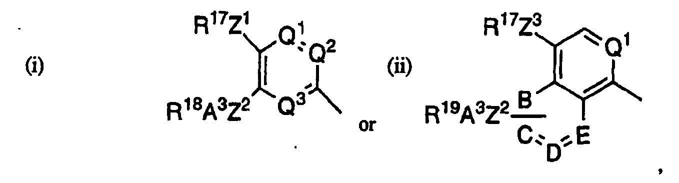 where R¹⁷ is a straight- or branched-chain alkyl group of 1 to about 6 carbon atoms, optionally substituted by one or more halogen atoms, or when Z³ is a direct bond R¹⁷ may also represent a hydrogen atom, alkenyl or alkynyl group;

R¹⁸ represents an optionally substituted cycloalkyl, cycloalkenyl, heterocycloalkyl, aryl, partially saturated bicycloaryl or heteroaryl group;

R¹⁹ represents R²⁰, -OR²⁰, -SR²⁰, -SOR²⁰ ,-SO₂R²⁰, -SO₂NR²⁰R²¹, -NR²⁰SO₂R²¹, -NR²⁰R²¹, -O(C=O)NR²⁰R²¹, -NR²⁰C(=O)R²¹, -N(OH)C(=O)R²⁰, or -C(=O)N(OH)R²¹, where R²⁰ and R²¹, which may be the same or different, each represent a hydrogen atom, or an alkyl, alkenyl, heterocycloalkyl, cycloalkyl, cycloalkenyl, aryl, heteroaryl, arylalkyl or heteroarylalkyl group, or the group NR²⁰R²¹ represents a 5 to 7 membered cyclic amine optionally containing one or more additional heteroatom selected from O, N, or S), wherein where R¹⁸ represents a substituted cycloalkyl group, the cycloalkyl group is substituted by one or more (e.g. 1,2 or 3) substituents chosen from OR²³, SR²⁴, SOR²⁴, SO₂R²⁴, NH₂, NR¹³R²⁴, =NOR²⁴, =NOH, =NNHR²⁴, =NOCONHR²⁴, =NCO₂R²⁴, SOR²⁴, NHCOR²⁴, NHSO₂R²⁴ SO₂NR¹³R²⁴, R²³, CONHR²⁴, CONHCH₂CO₂R¹³, CONR²⁴R¹³, or N₃; wherein R²³ is hydrogen, alkyl, cycloalkyl, aryl, arylallryl, heteroaryl or heteroarylalkyl; R²⁴ is alkyl, cycloalkyl, cycloalkenyl, heterocycloalkyl, aryl, arylalkyl, heteroaryl or heteroarylalkyl; or the substituent NR¹³R²⁴ forms a 4-7 membered cyclic amine which optionally contains an additional heteroatom selected from O, S, NH or NR¹³, and wherein R¹⁸ represents a substituted hetenocycloallcyl group containing a nitrogen atom the ring is substituted on one or more (e.g. 1, 2 or 3) of the ring carbon atoms and the substituents are chosen from oxo, cyano, CO₂R¹³, CONHCH₂CO₂R¹³, aryl, arylalkyl, alkyl or hydroxyalkyl, and/or is substituted on the ring nitrogen atom and the substituent is chosen from R¹³, (CH₂)ₙCO₂H, (CH₂)ₙCO₂R²⁴ (CH₂)ₙCONR¹³R²⁴, (CH₂)ₙCOR²⁴, CONH₂, CONHR²⁴, COR²⁴, SO₂R²⁴, or OR²⁴;

A³ represents a direct bond, a straight- or branched C₁₋₆alkylene chain optionally substituted by halogen, hydroxyl, alkoxy, oxo, cycloalkyl, aryl or heteroaryl; or A³ represents a straight- or branched-carbon chain comprising from 2 to about 6 carbon atoms which contains a double or triple carbon-carbon bond, or is interrupted by an oxygen or sulfur atom, a phenylene, imino (-NH-) or alkylimino linkage, or a sulfinyl or sulfonyl group,

Z¹ and Z³ each represents an oxygen or sulfur atom, a direct bond or NH;

Z² represents an oxygen or sulfur atom, or a direct bond;

B, C, D, and E independently represent a carbon or heteroatom selected from O, S, N, NOR²² or NR²² where R²² is hydrogen or a C₁₋₄straight- or branched-chain alkyl aryl, arylC₁₋₄alkyl, heteroaryl or hteteroaryl C₁₋₄ alkyl group, or three of B, C, D or E represent a carbon or heteroatom selected from O, N, NR²², or S and the other represents a direct bond; but excluding compounds where two O or S atoms are in adjacent positions, and the bonds joining B, C, D and E may be single or double bonds;

Q¹, Q² and Q³, which may be the same or different, each represents a CH or CX¹ linkage or a nitrogen atom; and X¹ represents a halogen atom; and

n is 0, 1 or 2; or

an N-oxide thereof, prodrug thereof, acid isostere there of, pharmaceutically acceptable salt, thereof, or solvate thereof,
comprising treating a polymeric hydroxamic acid resin compound of formula 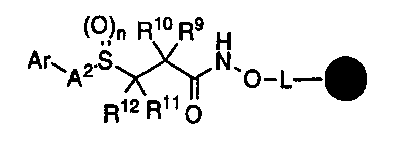 with acid.

In another aspect, this invention is directed to a process for the preparation of a polymeric oxime ether resin compound of formula 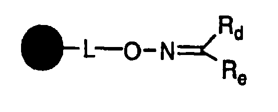 wherein 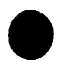 and L are as defined herein is and R_{d} and R_{c} are independently H, aliphatic or aromatic, comprising reacting a polymeric hydroxylamine resin compound of formula 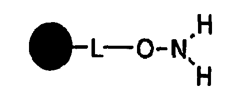 with a carbonyl compound of formula

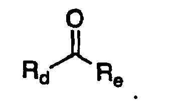

In another aspect, this invention is directed to a process for the preparation of an α-amine compound of formula 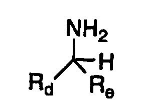 wherein R_{d} and R_{c} are independently H, aliphatic or aryl, provided that R_{d} and R_{c} are not both H, comprising reductively cleaving a polymeric oxime ether resin compound of formula 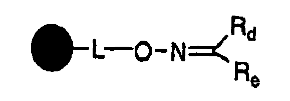 wherein 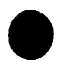 and L are as defined herein.

In another aspect, this invention is directed to a process for the preparation of a substituted α-amine compound of formula 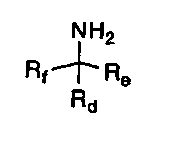 wherein R_{d} and R, are independently H, aliphatic or aomatic, provided that R_{d} and R_{c} are not both H,and-R_{f} is aliphatic or aromatic, comprising
(a) reacting a polymeric oxime ether compound of formula 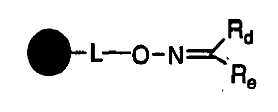 wherein 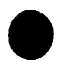 and L are as defined herein, with an organometallic reagent of formula R_{f}M wherein Rf is an aliphatic or aromatic anion and M is a metal cation, to form a polymeric α-substituted hydroxylamine resin compound of formula 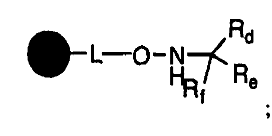 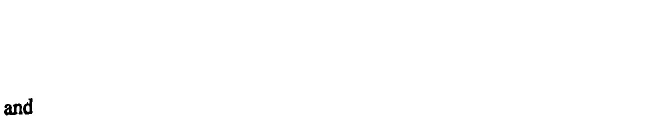
(b) reductively cleaving the α-substituted hydroxylamine resin compound.

In another aspect, this invention is directed to a process for the preparation of a lactone compound of formula 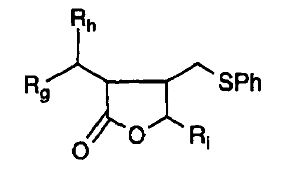 wherein R_{g}, Rₕ and R, are aliphatic or aromatic and Ph is phenyl, comprising
(a) treating an α,β-unsaturated polymeric hydroxamic acid ester resin compound of formula 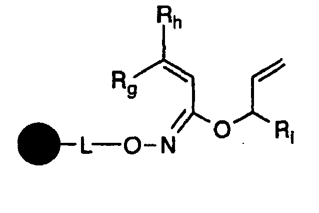 wherein 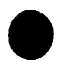 and L are as defined herein, with thiophenol and a radical initiator to form a polymeric oximyl lactone compound of formula 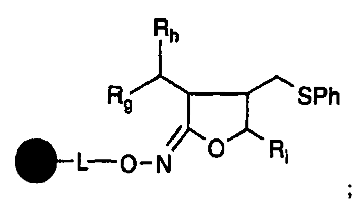 and
(b) treating the polymeric oximyl lactone compound with aqueous acid.

In another aspect, this invention is directed to a process for the preparation of an α,β-unsaturated polymeric hydroxamic acid ester resin compound of formula 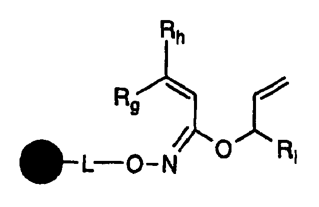 wherein 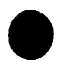 ,L and R, and Rₕ and Rₜ are as defined herein, comprising reacting a polymeric hydroxylamine resin compound of formula 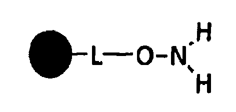 with an α,β-unsaturated carboxylic acid ester compound of formula

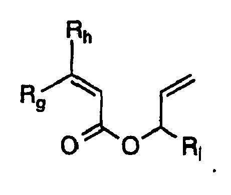

In another aspect, this invention is directed to a process for the preparation of an α-cyclic hydroxylamine compound of formula 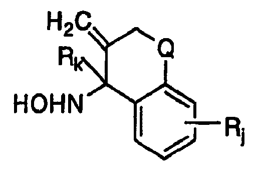 wherein Rⱼ and Rₖ are aliphatic or aromatic and Q is -O- or -CH2-, comprising
(a) treating a polymeric acetophenone oxime compound of formula 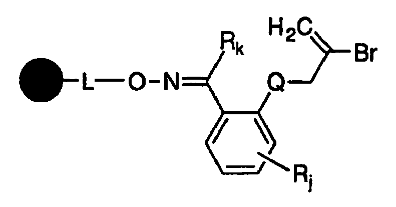 wherein 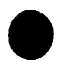 and L are as defined herein, with trialkyltin hydride and a radical initiator to form a polymeric α-cyclic hydroxylamine resin compound of formula 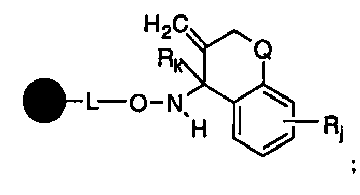 and
(b) treating the polymeric α-cyclic hydroxylamine resin compound with aqueous acid.

In another aspect, this invention is directed to a process for the preparation of a α-cyclic amino compound of formula 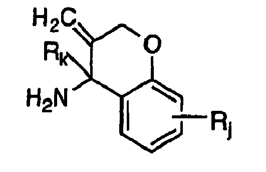 wherein Rⱼ and Rₖ are aliphatic or aromatic and Q is -O- or -CH₂-, comprising reductively cleaving a a polymeric α-cyclic hydroxylamine resin compound of formula 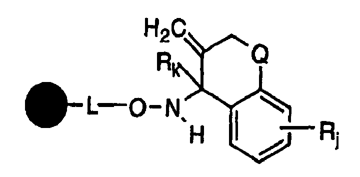 wherein 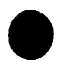 and L are as defined herein.

In another aspect, this invention is directed to a process for the preparation of a α-cyclic hydroxylamine compound of formula 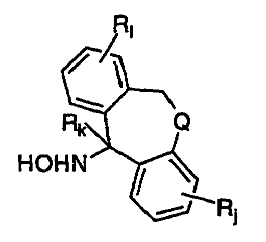 wherein Rⱼ, Rₖ and Rₗ, are aliphatic or aromatic and Q is -O- or -CH₂-, comprising
(a) treating a polymeric acetophenone oxime compound of formula 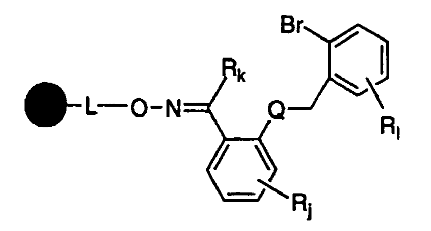 wherein 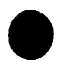 and L are as defined herein, with trialkyltin hydride and a radical initiator to form a polymeric α-cyclic hydroxylamine resin compound of formula 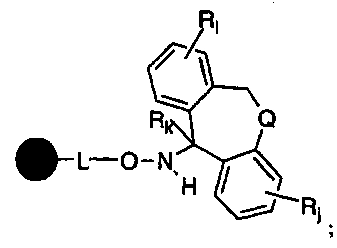 and
(b) treating the polymeric α-cyclic hydroxylamine resin compound with aqueous acid.

In another aspect, this invention is directed to a process for the preparation of a α-cyclic amino compound of formula 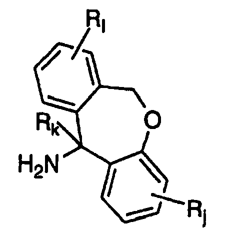 wherein R₁, Rₖ and Rₗ, are aliphatic or aromatic and Q is -O- or -CH₂-, comprising reductively cleaving a a polymeric α-cyclic hydroxylamine resin compound of formula 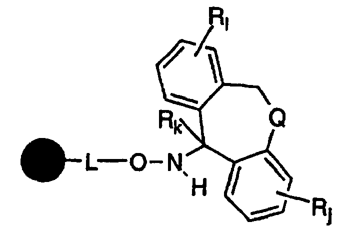 wherein 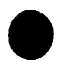 and L are as defined herein.

In another aspect, this invention is directed to a N-protected hydroxylamine resin compound of formula 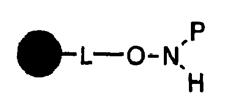 wherein
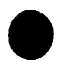 and L are as defined herein and P is an amine protecting group, provided that P is other than 4-methoxybenzy] or 2,4-dimethoxybenzyl.

In another aspect, this invention is directed to a polymeric tetrafluorophenyl hydroxylamine resim compound of formula 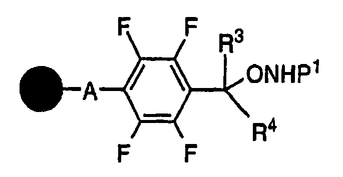 wherein 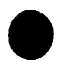 A, R' and R⁴ are as defined herein and P' is an amine protecting group.

### DETAILED DESCRIPTION OF THE INVENTION

### Definitions of Terms

As used above, and throughout the description of the invention, the following terms, unless otherwise indicated, shall be understood to have the following meanings.

"Solid support" means a substrate which is inert to the reagents and reaction conditions described herein, as well as being substantially insoluble in the media used. Representative solid supports include inorganic substrates such as kieselguhr, silica gel, and controlled pore glass; organic polymers including polystyrene, including 1-2% copolystyrene divinyl benzene (gel form) and 20-40% copolystyrene divinyl benzene (macro porous form), polypropylene, polyethylene glycol, polyacrylamide, cellulose, and the like; and composite inorganic/polymeric compositions such as polyacrylamide supported within a matrix of kieselguhr particles. *See* J.M. Stewart and J.D. Young, *Solid Phase Peptide Synthesis,* 2nd. Ed., Pierce Chemical Co. (Chicago, IL, 1984).

In addition,"solid support" includes a solid support as described above which is affixed to a second inert support such as the pins described in Technical Manual, Multipin™ SPOC, Chiron Technologies (1995) and references therein which comprise a detachable polyethylene- or polyproylene-based head grafted with an amino functionalized methacrylate copolymer and an inert stem.

In addition, "solid support" includes polymeric supports such as the polyethylene glycol supports described by Janda et al., *Proc. Natl. Acad. Sci. USA,* 92, 6419-6423 (1995) and S. Brenner, WO 95/16918, which are soluble in many solvents but can be precipitated by the addition of a precipitating solvent.

"Polymeric hydroxylamine resin compound" means a solid support as defined above which is chemically modified as is known in the art to incorporate a plurality of hydroxylamine (-ONH₂) or protected hydroxylamine (-ONHP) groups. The hydroxylamine or protected hydroxylamine groups are covalently bound directly to the solid support or attached to the solid support by covalent bonds through a linking group. The polymeric hydroxylamine resin compounds according to the process aspect of this invention are designated herein as 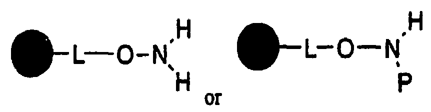 wherein 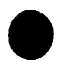 is a solid support as defined herein, L is absent or a linking group and P is an amine protecting group.

"Linking group" and "linker" mean a group through which the amino or aminomethyl functionality may be covalently linked to the solid support. The linking group is generally inert to the reagents and reaction conditions described herein.

"Amine protecting group" means an easily removable group which is known in the art to protect an amino group against undesirable reaction during synthetic procedures and to be selectively removable. The use of amine protecting groups is well known in the art for protecting against undesirable reactions during a synthetic procedure and many such protecting groups are known, cf, for example, T.H. Greene and P.G.M. Wuts, Protective Groups in Organic Synthesis, 2nd edition, John Wiley & Sons, New York (1991), incorporated herein by reference. Representative amine protecting groups include formyl, acetyl, chloroacetyl, trichloroacetyl, o-nitrophenylacetyl, o-nitrophenoxyacetyl, trifluoroacetyl, acetoacetyl, 4-chlorobutyryl, isobutyryl, o-nitrocinnamoyl, picolinoyl, acylisothiocyanate, aminocaproyl, benzoyl, methoxycarbonyl, 9-fluorenylmethoxycarbonyl (Fmoc), 2,2,2-trifluoroethoxycarbonyl, 2-trimethylsilylethxoycarbonyl (Teoc), vinyloxycarbonyl, allyloxycarbonyl, t-butyloxycarbonyl (BOC), 1,1-dimethylpropynyloxycarbonyl, benzyloxycarbonyl (Cbz), p-nitrobenzyloxycarbony, 2,4-dichlorobenzyloxycarbonyl, allyoxycarbonyl (Aloc), 2-(p-biphenyl)isopropyloxycarbonyl (Bpoc), adamantyloxycarbonyl (Adoc), 2-(3,5-dimethoxyphenyl)-propyl-2-oxycarbonyl (Ddz), t-butyl (t-Bu), p-methoxybenzyloxycarbonyl (Moz), p-nitrobenzyloxycarbonyl (4-NO₂-Z), 2-(phenylsulfonyl)ethoxycarbonyl, 2,4-dimethoxybenzyloxycarbonyl, o-nitrobenzyloxycarbonyl, 1-(2-oxo-1,2-diphenylethyl)methyloxycarbonyl, (1,3-dithione-2-yl)methoxycarbonyl (Dmoc), pyridylethyl (Pyoc), 4-nitrophenylallyloxycarbonyl (Noc), 2-nitro-4,5-dimethoxybenzyloxycarbonyl, dimethyl-t-butyl silyl o-nitrobenzylsulfonyl (o-Nbs), p-nitrobenzylsulfonyl (p-Nbs), 2-nitro-4-trifluoromethylbenzenesulfonyl, and the like.

"Natural amino acid" means a carboxylic acid compound having an amino group α to the carboxylate group, i.e, a compound of formula H₂N-CHR-CO₂H wherein R is aliphatic or aromatic as defined herein. Preferred amino acids have L stereochemistry at the α-carbon. The most preferred natural amino acids are the so-called natural α amino acids, ie alanine, valine, leucine, isoleucine, proline, phenylalanine, tryptophan, methionine, glycine, serine, threonine, cysteine, tyrosine, asparagine, glutamine, aspartic acid, glutamic acid, lysine, arginine and histidine.

"Peptide" and "polypeptide" mean a polymer in which the monomers are natural or unnatural amino acid residues joined together through amide bonds. The term "peptide backbone" means the series of amide bonds through which the amino acid residues are joined. The term "amino acid residue" means the individual amino acid units incorporated into the peptides or polypeptides.

"Unnatural amino acid" means a carboxylic acid compound having an amino group therein in a position other than a to the carboxylate group. Representative unnatural amino acids include β-alanine and γ-aminobutyric acid.

"Aliphatic" means a compound or radical which does not contain an aromatic ring. Representative aliphatic groups include alkyl, alkenyl, alkynyl, cycloalkyl, cycloalkenyl, and the like.

"Aromatic" means a compound or radical which contains at least one aromatic ring. The term "Aromatic ring" includes both aryl and heteroaryl rings as defined herein. Representative aromatic groups include aryl, arylalkenyl, arylalkyl, arylalkynyl, benzyl, heteroaryl, heteroarylaikenyl, heteroarylallcyl, heteroarylalkynyl, and the like.

"Acid bioisostere" means a group which has chemical and physical similarities producing broadly similar biological properties (see Lipinski, Annual Reports in Medicinal Chemistry, 1986,21,p283 "Bioisosterism In Drug Design"; Yun, Hwahak Sekye, 1993,33,p576-579 "Application Of Bioisosterism To New Drug Design"; Zhao, Huaxue Tongbao, 1995,p34-38 "Bioisosteric Replacement And Development Of Lead Compounds In Drug Design"; Graham, Theochem,1995,343,p105-109 "Theoretical Studies Applied To Drug Design:ab initio Electronic Distributions In Bioisosteres"). Examples of suitable acid bioisosteres include: -C(=O)-NH-OH, -C(=O)-CH₂OH, -C(=O)-CH₂SH, -C(=O)-NH-CN, sulpho, phosphono, alkylsulfonylcarbamoyl, tetrazolyl, arylsulfonylcarbamoyl, heteroarylsulfonylcarbamoyl, N-methoxycarbamoyl, 3-hydroxy-3-cyclobutene-1,2-dione, 3,5-dioxo-1,2,4-oxadiazolidinyl or heterocyclic phenols such as 3-hydroxyisoxazolyl and 3-hydoxy-1-methylpyrazolyl.

"Acyl" means an H-CO- or allcyl-C0. group in which the alkyl group is as defined herein. Preferred acyls contain a lower alkyl. Exemplary acyl groups include formyl, acetyl, propanoyl, 2-methylpropanoyl, butanoyl and palmitoyl.

"Acylamino" is an acyl-NH- group wherein acyl is as defined herein.

"Alkenyl" means an aliphatic hydrocarbon group containing a carbon-carbon double bond and which may be straight or branched having about 2 to about 15 carbon atoms in the chain. Preferred alkenyl groups have 2 to about 12 carbon atoms in the chain; and more preferably about 2 to about 4 carbon atoms in the chain. Branched means that one or more lower alkyl groups such as methyl, ethyl or propyl are attached to a linear alkenyl chain. "Lower alkenyl" means about 2 to about 4 carbon atoms in the chain which may be straight or branched. The alkenyl group may be substituted by one or more halo or cycloalkyl. Exemplary alkenyl groups include ethenyl, propenyl, n-butenyl, i-butenyl, 3-methylbut-2-enyl, n-pentenyl, heptenyl, octenyl, cyclohexylbutenyl and decenyl.

"Alkoxy" means an alkyl-O- group in which the alkyl group is as defined herein. Exemplary alkoxy groups include methoxy, ethoxy, n-propoxy, i-propoxy, n-butoxy and heptoxy.

"Alkoxyalkyl" means an alkyl-O-alkyl- group in which the alkyl groups are independently as defined herein. Exemplary alkoxy groups include methoxyethyl, ethoxymethyl, n-butoxymethyl and cyclopentylmethyloxyethyl.

"Alkoxycarbonyl" means an alkyl-O-CO- group in which the alkyl group is as defined herein. Exemplary alkoxycarbonyl groups include methoxy- and ethoxycarbonyl.

"Alkyl" means an aliphatic hydrocarbon group which may be straight or branched having about 1 to about 15 carbon atoms in the chain. Preferred alkyl groups have 1 to about 12 carbon atoms in the chain. Branched means that one or more lower alkyl groups such as methyl, ethyl or propyl are attached to a linear alkyl chain. "Lower alkyl" means about 1 to about 4 carbon atoms in the chain which may be straight or branched. The alkyl group may be substituted by one or more halo, cycloalkyl or cycloalkenyl. Exemplary alkyl groups include methyl, fluoromethyl, difluoromethyl, trifluoromethyl, cyclopmpylmethyl, cyclopentylmethyl, ethyl, n-propyl, i-propyl, *n*-butyl, *t*-butyl, *n*-pentyl, 3-pentyl, heptyl, octyl, nonyl, decyl and dodecyl. Preffered alkyl groups for R⁹ include methyl, fluoromethyl, difluoromethyl, trifluoromethyl, and ethyl.

"Alkylsulfinyl" means an alkyl-SO- group in which the alkyl group is as defined above. Preferred groups are those in which the alkyl group is lower alkyl.

"Alkylsulfonyl" means an alkyl-SO₂- group in which the alkyl group is as defined above. Preferred groups are those in which the alkyl group is lower alkyl.

"Alkylthio" means an alkyl-S- group in which the alkyl group is as previously described. Exemplary alkylthio groups include methylthio, ethylthio, i-propylthio and heptylthio.

"Alkynyl" means an aliphatic hydrocarbon group containing a carbon-carbon triple bond and which may be straight or branched having about 2 to about 15 carbon atoms in the chain. Preferred alkynyl groups have 2 to about 12 carbon atoms in the chain; and more preferably about 2 to about 4 carbon atoms in the chain. Branched means that one or more lower alkyl groups such as methyl, ethyl or propyl are attached to a linear alkenyl chain. Exemplary alkynyl groups include ethynyl, propynyl, n-butynyl, *i*-butynyl, 3-methylbut-2-ynyl, and *n*-pentynyl.

"Aroyl" means an aryl-CO- group in which the aryl group is as defined herein. Exemplary groups include benzoyl and 1- and 2-naphthoyl.

"Aroylamino" is an aroyl-NH- group wherein aroyl is as defined herein.

"Aryl" as a group or part of a group denotes an optionally substituted monocyclic or multicyclic aromatic carbocyclic moiety of about 6 to about 10 carbon atoms. Exemplary aryl include phenyl or naphthyl, or phenyl or naphthyl substituted with one or more aryl group substituents which may be the same or different, where "aryl group substituent" includes acyl, acylamino, alkoxy, alkoxycarbonyl, alkyl, alkylsulfinyl, alkylsulfonyl, alkylthio, aroyl, aroylamino, aryl, arylalkoxy, arylalkoxycarbonyl, arylalkyl, arylalkylthio, aryloxy, aryloxycarbonyl, arylsulfinyl, arylsulfonyl, arylthio, carboxy, cyano, halo, heteroaroyl, heteroaryl, heteroarylalkyl, heteroarylamino, heteroaryloxy, hydrogen, hydroxy, hydroxyalkyl, nitro, Y¹Y²N-, Y¹ Y²NCO- or Y¹Y²NSO₂-, where Y¹ and Y² are independently hydrogen, alkyl, arylalkyl, and aryl, or Y1 and Y2 taken together with the N atom through which Y1 and Y2 are linked form a 4-7 membered heterocylyl which optionally contains an additional heteroatom selected from O, S, NH or NR¹³. Preferred aryl group substituents include acyl, acylamino, alkoxycarbonyl, alkyl, alkylthio, aroyl, cyano, halo, hydrogen, hydroxy, nitro, Y¹Y²N-, Y¹ Y²NCO- or Y¹ Y²NSO₂-, where Y¹ and Y² are independently hydrogen and alkyl.

"Arylalkenyl" means an aryl-alkenyl- group in which the aryl and alkenyl are as previously described. Preferred arylalkenyls contain a lower alkenyl moiety. Exemplary arylalkenyl groups include styryl and phenylallyl.

"Arylalkyl" means an aryl-alkyl- group in which the aryl and alkyl are as previously described. Preferred arylalkyls contain a lower alkyl moiety. Exemplary arylalkyl groups include benzyl, 2-phenethyl and naphthlenemethyl.

"Arylallcyloxy" means an arylalkyl-O- group in which the arylalkyl group is as previously described. Exemplary arylalkyloxy groups include benzyloxy and 1- or 2-naphthalenemethoxy.

"Arylalkyloxyalkenyl" means an arylalkyl-O-alkenyl group in which the arylalkyl and alkenyl groups are as previously described. An exemplary arylalkyloxyalkenyl group is 3-benzyloxyallyl.

"Arylalkyloxyalkyl" means an arylalkyl-O-alkyl group in which the arylalkyl and alkyl groups are as previously described. An exemplary arylalkyloxyalkyl group is benzyloxyethyl.

"Arylalkoxycarbonyl" means an arylalkyl-O-CO- group. An exemplary arylalkoxycarbonyl group is benzyloxycarbonyl.

"Arylalkylthio" means an arylalkyl-S- group in which the arylalkyl group is as previously described. An exemplary arylallcylthio group is benzylthio.

"Arylalkynyl" means an aryl-alkynyl- group in which the aryl and alkynyl are as previously described. Preferred arylalkynyls contain a lower alkynyl moiety. An exemplary arylallcynyl group is phenylacetylenyl.

"Aryloxy" means an aryl-O- group in which the aryl group is as previously described. Exemplary aryloxy groups include phenoxy and naphthoxy.

"Aryloxyalkenyl" means an aryl-O-alkenyl- group in which the aryl or alkenyl groups are as previously described. An exemplary aryloxyalkenyl groups is phenoxyallyl.

"Aryloxyalkyl" means an aryl-O-alkyl- group in which the aryl or alkyl groups are as previously described. An exemplary aryloxyalkyl groups is phenoxypropyl.

"Aryloxycarbonyl" means an aryl-O-CO- group in which the aryl group is as previously described. Exemplary aryloxycarbonyl groups include phenoxy- and naphthoxycarbonyl.

"Arylsulfinyl" means an aryl-SO- group in which the aryl group is as previously described.

"Arylsulfonyl" means an aryl-SO₂- group in which the aryl group is as previously described.

"Arylthio" means an aryl-S- group in which the aryl group is as previously described. Exemplary arylthio groups include phenylthio and naphthylthio.

"(N-carbamoyl)cyclic amine" means a 5 to 7 membered cycloalkyl ring system where one of the ring carbon atoms is replaced by the group N-C(=O)-NH₂. An exemplary (N-carbamoyl)cyclic amine is N-carbamoylpiperidinyl.

"Benzyl" means a Phenyl-CH₂- group in which the phenyl ring is unsubstituted or substituted with one or more substituents independently selected from alkyl, alkoxy, halogen, and haloalkyl.

"Cycloallcenyl" means a non-aromatic monocyclic or multicyclic ring system containing a carbon-carbon double bond and having about 3 to about 10 carbon atoms. Preferred monocyclic cycloalkenyl rings include cyclopentenyl, cyclohexenyl or cycloheptenyl; more preferred is cyclopentenyl. A preferred multicyclic cycloalkenyl ring is norbornenyl. The cycloalkenyl group may be substituted by one or more halo, methylene (H₂C=) or alkyl.

"Cycloalkyl" means a non-aromatic mono- or multicyclic ring system of about 3 to about 10 carbon atoms. Exemplary monocyclic cycloalkyl rings include cyclopropyl, cyclopentyl, cyclohexyl and cycloheptyl. Preferred monocyclic cycloalkyl rings for R¹⁸ include cyclopentyl and cyclohexyl. A preferred monocyclic cycloalkyl rings for R¹⁹ is cyclopropyl. Exemplary multicyclic cycloalkyl rings include perhydronaphthyl, adamant-(1- or 2-)yl and norbomyl and spirobicyclic groups, e.g. spiro[4,4]non-2-yl. The cycloalkyl group may be substituted by one or more halo, methylene (H₂C=) or alkyl groups. Preferred moncyclic cycloalkyl rings for R¹⁸ include cyclopentyl and cyclohexyl.

### Preferred cycloalkyl groups for R¹⁹ include cyclopropyl.

"Cycloalkyoxy" means an cycloalkyl-O- group in which the cycloalkyl group is as previously described. Exemplary cycloalkoxy groups include cyclopentyloxy and cyclohexyloxy.

"Cyclocarbamoylalkyl" means a compound of formula 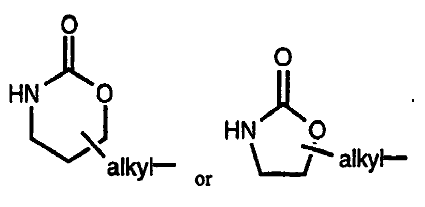 in which the cyclocarbamoyl group consists of the oxooxazaheterocyclyl ring moiety, and the alkyl group is as previously described. The alkyl moiety may be attached to the carbamoyl through either a carbon atom or the nitrogen atom of the carbamoyl moiety. An exemplary cyclocarbamoylalkyl group is N-oxazolidinylpropyl.

"Cycloimidyl" means a compound of formula 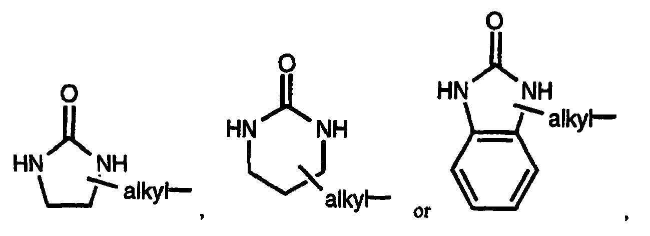 in which the imide group consists of the oxodiazaheterocyclyl ring moiety, and the alkyl group is as previously described. The alkyl moiety may be attached to the carbamoyl through either a carbon atom or nitrogen atom of the carbamoyl moiety. An exemplary imidealkyl group is N-phthalimidepropyl.

"Halo" and "halogen" mean fluoro, chloro, bromo, or iodo. Preferred are fluoro, chloro or bromo, and more preferred are fluoro or chloro.

"Haloalkyl" means an alkyl group as defined herein substituted with one or more halogen atoms. Representative haoloalkyl groups include chloromethyl, bromoethyl, trifluoromethyl, and the like.

"Heteroaroyl" means a heteroaryl-CO- group in which the heteroaryl group is as defined herein. An exemplary heteroaroyl group is pyridylcarbonyl.

"Heteroaryl" as a group or part of a group denotes an optionally substituted aromatic monocyclic or multicyclic hydrocarbon ring system of about 5 to about 10 atoms in which one or more of the ring members is/are element(s) other than carbon, for example nitrogen, oxygen or sulphur. The "heteroaryl" may also be substituted by one or more aryl group substituents. Examples of suitable optionally substituted heteroaryl groups include furyl, isoxazolyl, isoquinolinyl, isothiazolyl, 1,2,3-oxadiazole, 1,2,4-oxadiazole, 1,2,5-oxadiazole, 1,3,4-oxadiazole,pyrazinyl, pyridazinyl, pyridyl, pyrimidinyl, quinolinyl, 1,3,4-thiadiazolyl, thiazolyl, thienyl, and 1,2,3- and 1,2,4-triazolyl groups, optionally substituted by one or more aryl group substituents as defined above.
When R¹⁸ or R¹⁹ contains an optionally substituted heteroaryl group this may particularly represent an optionally substituted "azaheteroaryl" group (where the term "azaheteroaryl" means a heteroaryl group of about 5 to about 10 ring members in which one or more of the ring members is/are nitrogen). Optional substituents for the heteroaryl group within R¹⁸ or R¹⁹ include, for example, halogen atoms and alkyl, aryl, arylalkyl, hydroxy, oxo, hydroxyalkyl, haloallcyl (for example trifluoromethyl), alkoxy, haloalkoxy (for example trifluoromethoxy), aryloxy, and arylallryloxy groups. Preferred heteroaryl groups within R¹⁸ include optionally substituted thienyl, thiazolyl, pyridyl, 1,2,4-oxadiazole or 1,3,4-oxadiazole. A preferred heteroaryl groups within R¹⁹ is optionally substituted pyridyl

"Heteroarylalkenyl" means an heteroaryl-alkenyl- group in which the heteroaryl and alkenyl are as previously described. Preferred heteroarylalkenyls contain a lower alkenyl moiety. An exemplary heteroarylalkenyl group is 4-pyridylvinyl.

"Heteroarylalkyl" means an heteroaryl-alkyl- group in which the heteroaryl and alkyl are as previously described. Preferred heteroarylalkyls contain a lower alkyl moiety. An exemplary heteroarylalkyl group is 4-pyridylmethyl.

"Heteroarylalkyloxy" means an heteroarylalkyl-O- group in which the heteroarylalkyl group is as previously described. An exemplary heteroarylalkyloxy group is 4-pyridylmethyloxy.

"Heteroarylalkyloxyalkenyl" means an heteroarylalkyl-O-alkenyl group in which the heteroarylalkyl and alkenyl groups are as previously described. An exemplary heteroarylalkyloxyalkenyl group is 4-pyridylmethyloxyallyl.

"Heteroarylalkyloxyalkyl" means an heteroarylalkyl-O-alkyl group in which the heteroarylalkyl and alkyl groups are as previously described. An exemplary heteroarylalkyloxy group is 4-pyridylmethyloxyethyl.

"Heteroarylalkynyl" means an heteroaryl-alkynyl- group in which the heteroaryl and alkynyl are as previously described. Preferred heteroarylalkynyls contain a lower alkynyl moiety. An exemplary heteroarylalkynyl group is 4-pyridylethynyl.

"Heterocyclyl" means an about 4 to about 10 member monocyclic or multicyclic ring system wherein one or more of the atoms in the ring system is an element other than carbon chosen amongst nitrogen, oxygen or sulfur. The heterocyclyl may be optionally substituted by one or more alkyl group substituents. Exemplary heterocyclyl moieties include quinuclidine, pentamethylenesulfide, tetrahydropyranyl, tetrahydrothiophenyl, pyrrolidinyl, piperidinyl or tetrahydrofuranyl.

"Heterocyclylalkyl" means an heterocyclyl-alkyl- group in which the heterocyclyl and alkyl are as previously described. Preferred heterocyclylalkyls contain a lower alkyl moiety. An exemplary heteroarylalkyl group is tetrahydropyranylmethyl.

"Heterocyclylalkyloxyalkyl" means an heterocyclyl-alkyl-O-alkyl- group in which the heterocyclyl and alkyls groups independently are as previously described. An exemplary heteroarylalkyl group is tetrahydropyranyl-methyloxymethyl.

"Hydroxyalkyl" means a HO-alkyl- group in which alkyl is as previously defined. Preferred hydroxyallryls contain lower alkyl. Exemplary hydroxyalkyl groups include hydroxymethyl and 2-hydroxyethyl. "Partially saturated bicycloaryl" means a group in which an aryl and a cycloalkyl group are fused together to form a bicyclic structure. Exemplary arylalkyl groups include indanyl and tetrahydronaphthyl, especially indanyl.

### Preferred Embodiments

A process for the preparation of aldehydes and ketones according to this invention is outlined in Scheme 1 wherein Rₐ and R_{b} independently represent any aliphatic or aromatic group amenable to the solvents and reagents utilized in the processes described herein. The groups Rₐ and R_{b} may be further substituted and may contain functional groups suitable for further chemical transformations while attached to the hydroxylamine resin. It is understood that when these functional groups possess reactivity such that they could potentially interfere with the reactions described below, such functional groups should be suitably protected. For a comprehensive treatise on the protection and deprotection of common functional groups see T.H. Greene and P.G.M. Wuts, Protective Groups in Organic Synthesis, 2nd edition, John Wiley & Sons, New York (1991), incorporated herein by reference. R_{c} represents any aliphatic or aromatic group amenable for use as an organometallic reagent.

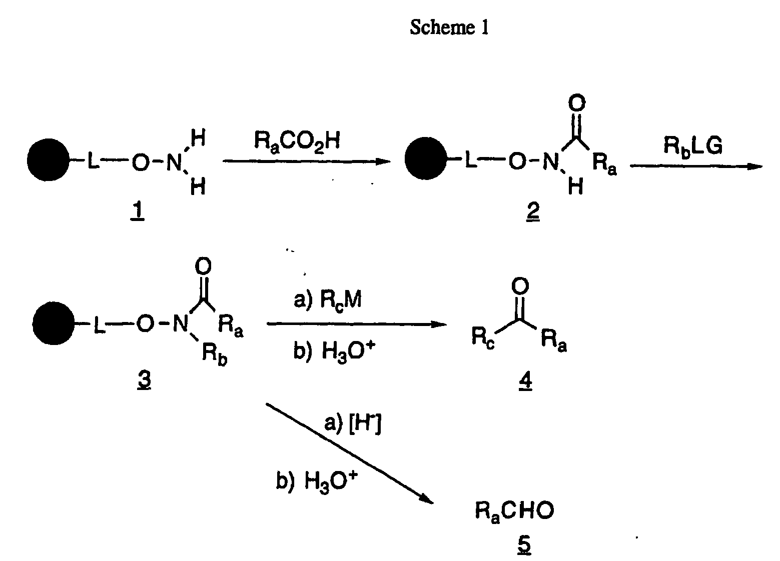

According to the foregoing Scheme 1, a polymeric hydroxylamine resin compound 1 is coupled with a carboxylic acid derivative of formula RₐCO₂H to form the polymeric hydroxamic acid resin compound 2. The coupling reaction is accomplished in the presence of an activating agent as is known in the art of peptide synthesis. Representative activating agents include isopropyl chloroformate, diisopropylcarbodiimide (DIC), 1-(3-dimethylaminopropyl)-3-ethylcarbodiimide (EDC), 1-hydroxybenzotriazole (HOBT), bis(2-oxo-3-oxazolidinyl)-phosphonic chloride (BOP-Cl), benzotriazole-1-yloxy-tris((dimethylamino)phosphonium)hexafluorophosphate (BOP), benzotriazole-1-yloxy-tris-pyrrolidino-phosphonium hexafluorophosphate (PyBROP), bromo-tris-pyrrolidino-phosphonium hexafluorophosphate (PyBOP), 2-(1H-benzotriazole-1-yl)-1.1.3.3-tetramethyluronium tetrafluoroborate (TBTU), 2-(1H-benzotriazole-1-yl)-1.1.3.3-tetramethyluronium hexafluoroborate (HBTU), 2-[2-oxo-1-(2H)-pyridyl]-1,1,3,3-bispentamethyleneuronoium tetrafluoroborate (TOPPipU), N,N'-dicyclohexylcarbodiimide (DCC), and the like. Suitable solvents for the coupling reaction include dichloromethane, DMF, DMSO, THF, and the like. Coupling times range from about 2 to about 24 hours, depending upon the resin and carboxylic acid derivative to be coupled, activating agent, solvent and temperature. The coupling is accomplished at from about -10 °C to about 50 °C, preferably at about ambient temperature.

The coupling reaction is preferably accomplished at ambient termperature in DMF using 1-(3-dimethylaminopropyl)-3-ethylcarbodiimide hydrochloride over about 12 hours.

The polymeric hydroxamic acid resin compound 2 is then alkylated with an alkylating agent of formula R_{b}LG, where LG is a leaving group, in the presence of a non-nucleophilic base such as 1,8-diazabicyclo[5.4.0]undec-7-ene (DBU) in an inert organic solvent such as toluene to form the N-alkylated polymeric hydroxamic acid resin compound 3. The alkylating agent RbLG may be added in an equimolar amount to an excess of to about 25 molar equivalents. About 15 molar equivalents is preferred. The non-nucleophilic base may be added in an equimolar amount to an excess of to about 10 molar equivalents. About 5 molar equivalents is preferred. The leaving group LG is any group amendable to nucleophilic displacement by the nitrogen atom of the polymeric hydroxamic acid resin compound 2 under the reaction conditions described above. A preferred leaving group is halogen. A sample of the the N-allcylated polymeric hydroxamic acid resin compound 3 may be subjected to acidolysis to cleave the substituted hydroxamic acid to confirm that the reaction proceeded satisfactorily.

Reaction of the polymeric N-alkylated hydroxamic acid resin compound 3 with an organometallic reagent of formula R_{c}M, wherein R_{c} is an aliphatic or aromatic anion and M is a metal cation, followed by acid hydrolysis provides the ketone 4. Preferred organometallic reagents are organolithium reagens of formula R_{c}Li and Grignard reagents of formula R_{c}MgX wherein X is halogen. In a preferred preparation of ketones according to this aspect of the invention, the polymeric N-alkylated hydmxamic acid resin compound 3 is treated with RcMgX in diethyl ether at ambient temperature over about 18 hours, and the reaction mixture is then quenched by addition of aqueous HCl or aqueous KHSO₄ to liberate the ketone 4.

Aldehydes are prepared by treatment of the the polymeric N-alkylated hydroxamic acid resin compound 3 with a hydride reducing agent, followed by acid hydrolysis as shown in Scheme 1 above. Representative hydride reducing agents include LiAlH₄, (*iso*-Bu)₂AlH, LiAlH(O-*t*-Bu)₃, LiAlH₄-EtOH, LiAlH₄-MeOH, and the like. Preferred reducing agents are LiAlH₄ and LiAlH₄-MeOH. The acid hydrolysis is preferably accomplished aqueous KHSO₄.

As shown in Scheme 1, the N-alkylated polymeric hydroxamic acid resin compound 3 is a Weinreb-like amide useful for the synthesis of aldehydes and ketones (S. Nahm and S. Weinreb, *Tet Lett.* 1981, 22, 3815-3818). This N-alkylated polymeric hydroxamic acid resin compound has advantages over the previous examples of resin bound Weinreb-like amides (J.-A. Fehrentz, M. Paris, A. Heitz, J. Velek, C.-F. Liu, F. Winternitz, and J. Martinez *Tet. Lett.,* 1995, *36,* 7871-7874. b) T. Q. Dinh and R. W. Armstrong, *Tet. Lett.,* 1996, *37,* 1161-1164) in that it can be N-alkylated with bulky lipophilic groups such as benzyl, substituted benzyl, naphthyl or any alkyl group necessary to optimize the reaction on the solid phase. The N-benzyl-O-methylpolystyrenyl moiety, for example, is well suited to form a stable metal chelated intermediate. The lipophilic benzyl group is believed to help shield the chelate adding to its stability.

A preferred process for the preparation of aldehydes and ketones is outlined in Scheme 2. In Scheme 2, "P" designates an amine protecting group as defined herein.

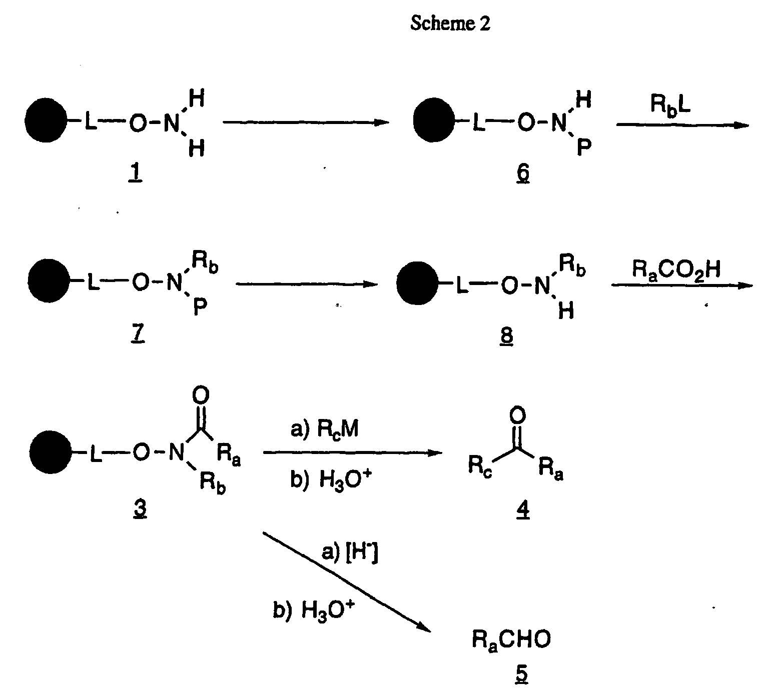

As shown in Scheme 2 above, the polymeric hydroxylamine resin compound is protected with an amine protecting group to form the N-protected polymeric hydroxylamine resin compound 6. The N-protected polymeric hydroxylamine resin compound 6 is then alkylated as described in Scheme 1 above to form the N-alkylated N-protected polymeric hydroxylamine resin compound 7. Removal of the amine protecting group provides the mono N-alkylated polymeric hydroxylamine resin compound 8. Coupling of 8 with a carboxylic acid compound of formula RₐCO₂H as described above provides the polymeric N-alkylated hydroxamic acid resin compound 3, which is converted to ketone 4 or aidehyde 5 as described in Scheme 1 above.

Preferred amine protecting groups include allyloxycarbonyl (Aloc), benzyloxycarbonyl (Cbz), p-methoxybenzyloxycarbonyl (Moz), p-nitrobenzyloxycarbonyl (4-NO₂-Z), trimethylsilylethoxycarbonyl (Teoc), 2,4-dimethoxybenzyloxycarbonyl, o-nitrobenzyloxycarbonyl, o-nitrobenzylsulfonyl (o-Nbs), p-nitrobenzylsulfonyl (p-Nbs), and 2-nitro-4-trifluoromethylbenzenesulfonyl.

The most preferred amine protecting group is allyloxycarbonyl.

Preferred polymeric N-protected hydroxylamine resin compounds include
N-allyloxycarbonyl-4-(O-methylhydroxylamine)phenoxymethyl-copoly(styrene-1% divinylbenzene) resin,
N-allyloxycarbonyl-4-[4-(O-methylhydroxylamine)-3-methoxyphenoxy]-(N-4-methylbenzhydryl)-butyramide-copoly(styrene-1 %-divinylbenzene)-resin,
N-allyloxycarbonyl-4-(2',4'-dimethoxyphenyl-O-methylhydroxylamine)-phenoxymethyl-copoly(styrene-1% divinylbenzene) resin,
N-allyloxycarbonyl-4-[4-(1-aminoxyethyl)-2-methoxy-5-nitrophenoxy]-(N-4-methylbenzhydryl)-butyramide-copoly(styrene-1 % divinylbenzene) resin,
N-allyloxycarbonyl-O-hydroxylamine-2'-chlorotrityl-copolystyrene-1 %-divinylbenzene-resin,
N-allyloxycarbonyl-O-hydroxylamine-trityl-copolystyrene-1 %-divinylbenzene-resin,
N-allyloxycarbonyl-5-(4-O-methylhydroxylamine -3,5-dimethoxyphenoxy)-valeric acid-copolystyrene-1%-divinyl benzene resin,
N-allyloxycarbonyl-4-O-methylhydroxylamine-3-methoxyphenoxy-copolystymne-1%-divinyl benzene resin,
N-allyloxycarbonyl4-(O-methylhydroxylamine)-2,3,5,6-tetrafluorophenoxymethyl-copoly(styrene-l% divinylbenzene) resin,
N-allyloxycarbonyl-4-(2',4'-dimethoxyphenyl-O-methylhydroxylamine)-2,3,5,6-tetrafluorophenoxymethyl-copoly(styrene-1 % divinylbenzene) resin and
N-allyloxycarbonyl-3-hydroxy-xanthydrolamine-copolystryene-1%-divinylbenzene resin.

The most preferred polymeric N-protected hydroxylamine resin compound is N-allyloxycarbonyl-4-(O-methylhydroxylamine)phenoxymethyl-copoly(styrene-1% divinylbenzene) resin.

When the carboxylic acid compound RₐCO₂H represents a natural or unnatural amino acid or peptide, the procedures described in Schemes 1 and 2 above present a facile route to the heretofore difficult to obtain amino acid or peptide aldehyde compounds.

A process for the preparation of amines according to this invention is outlined in Scheme 3. In Scheme 3, R_{d} and R_{c} independently represent H or any aliphatic or aromatic group amenable to the solvents and reagents utilized in the processes described herein, provided that R_{d} and R_{c} are not both H. The groups R_{d}, R_{b} and R_{c} may be further substituted and may contain functional groups suitable for further chemical transformations while attached to the hydroxylamine resin. It is understood that when these functional groups possess reactivity such that they could potentially interfere with the reactions described below, such functional groups should be suitably protected. For a comprehensive treatise on the protection and deprotection of common functional groups see T.H. Greene and P.G.M. Wuts, Protective Groups in Organic Synthesis, 2nd edition, John Wiley & Sons, New York (1991), incorporated herein by reference. R_{f} represents any aliphatic or aromatic group which is amenable for use as an organometallic reagent.

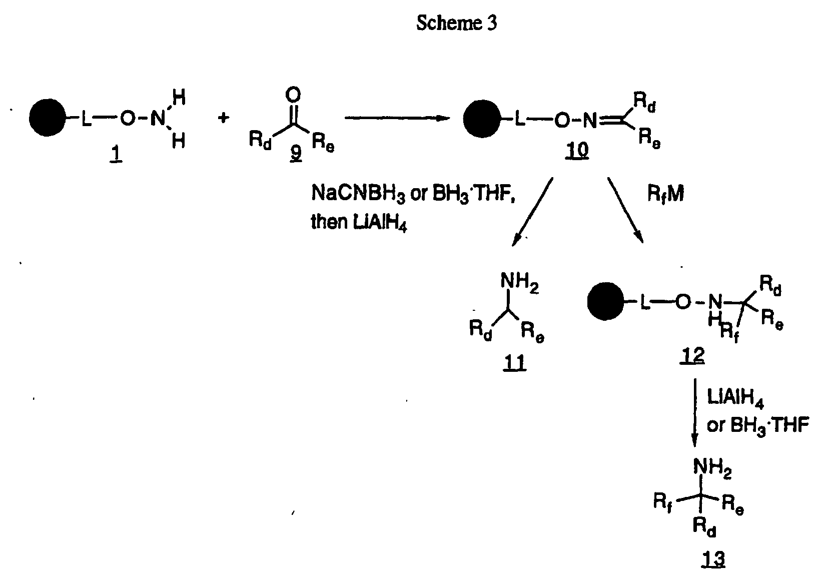

According to the foregoing Scheme 3, reaction of the polymeric hydroxylamine resin compound 1 with an aldehyde or ketone 9 provides the polymeric oxime ether resin compound 10. Oxime formation is preferably accomplished at about ambient temperature by swelling the polymeric hydroxylamine resin compound I in a suitable inert organic solvent such as dichloromethane, followed by addition of an excess of aldehyde or ketone. Reductive cleavage of the resin, for example by reaction with NaCNBH₃, or BH₃ THF, followed by LiA1H, provides the amine 11. Reaction of the polymeric oxime ether resin compound 10 with an organometallic reagent of formula R_{f}M, wherein Rᵣ is an aliphatic or aromatic anion and M is a metal cation as defined herein, provides the polymeric α-substituted hydroxylamine resin compound 12. Cleavage of the α-amine 13 from the resin, is accomplished, for example, using BH₃-THF or LiAlH₄. See Y. Ukaji et al., Chem. *Lett.,* 173, (1991) and R. P. Dieter et al., Can. J. *Chem.* 71, 814 (1993). Preferred metal cations are Li and MgX wherein X is halogen. With the aid of a chiral auxiliary such as a chiral benzyl hydroxyl amine linker, chiral α-substituted amines will result.

A process for the preparation of lactones via radical cyclization is shown in Scheme 4. In Scheme 4, R_{g}, Rₕ and R, are aliphatic or aryl as defined herein.

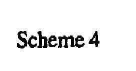

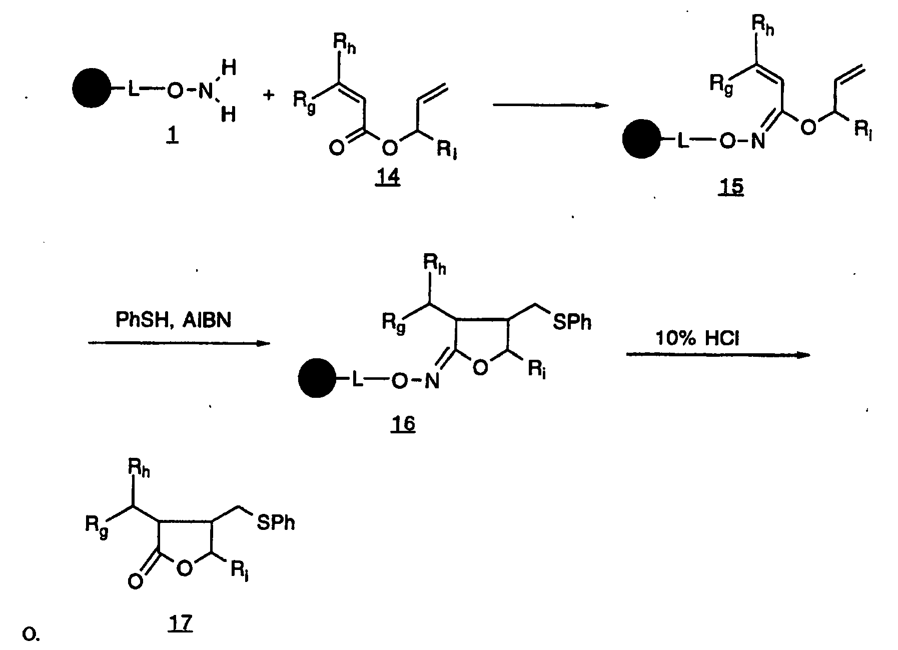

O. As shown in the foregoing Scheme 4, the polymeric hydroxylamine resin compound 1 is reacted with the α,β unstaurated carboxylic acid ester compound 14 to form the polymeric oximyl resin compound 15. Radical cyclization of 15, for example by heating in the presence of 2,2'-azobisisobutyronitrile (AIBN) and thiophenol in an inert organic solvent such as benzene results in formation of the polymeric g-lactone resin compound 16. Acid hydrolysis of 16, using, for example 10% aqueous HCl, provides the lactone 17. *See* O. Miyata et al., *Tet. Lett.,* 37, 229-232, (1996).

A process for the preparation of carbocyclic or heterocylic compounds by radical cyclization is shown in Scheme 5. In Scheme 5, Rⱼ, Rₖ and Rₗ, are aliphatic or aryl as defined herein. The methodology described in Scheme 1 is applicable to the preparation of 5-, 6- or 7-membered rings. Carbocyles result when the phenolic oxygen atom is replaced with a carbon atom.

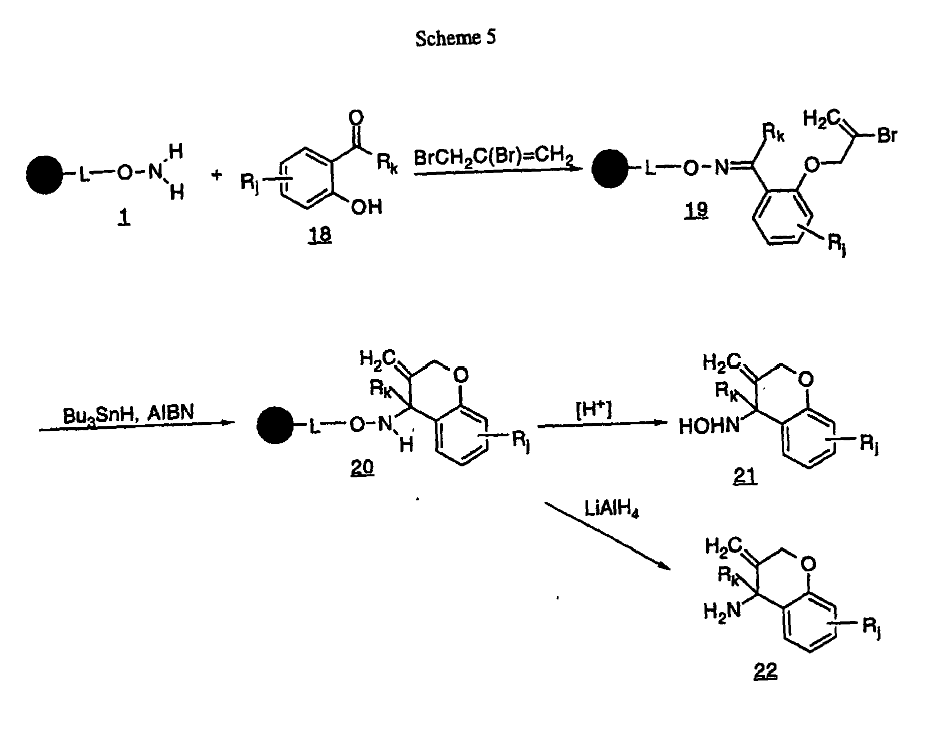

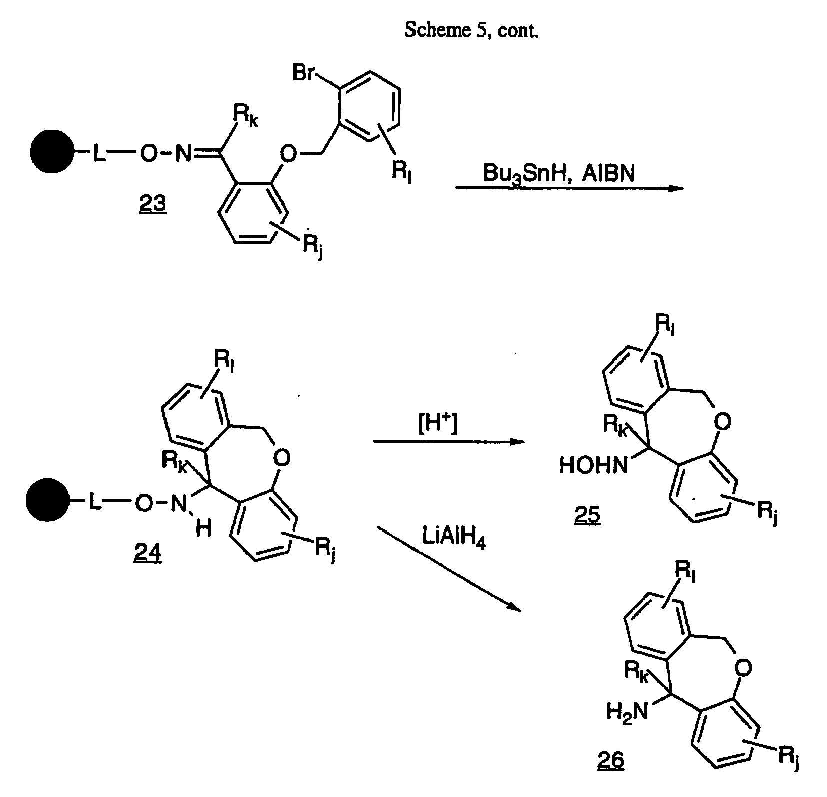

According to the foregoing Scheme 5, the polymeric hydroxylamine resin compound 1 is reacted with the acetophenone compound 18 and a bromoalkene compound or o-bromobenzyl compound to form the polymeric acetophenone oxime compounds 19 or 23. Radical cyclization of 19 or 23, for example by heating in the presence of AIBN and tri-n-butyltin hydride in an inert organic solvent such as benzene results in formation of the polymeric N-cyclyl hydroxylamine resin compounds 20 or 24. Treatment of 20 or 24 with acid, preferably trifluoroacetic acid, results in formation of the cyclic hydroxamic acid compounds 21 or 25. Reductive cleavage of 20 or 24, for example using LiAlH₄ as described in Scheme 3 above, results in formation of the cyclic amine compounds 22 or 26. *See* S.E. Booth et al., J. *Chem. Soc. Commun.,* 1248-1249, (1991).

A process for the preparation of a hydroxamic acid compound of formula 29, wherein n and the groups Ar, A², R⁹, R¹⁰, R¹¹ and R¹² are as defined above is shown in Scheme 6.

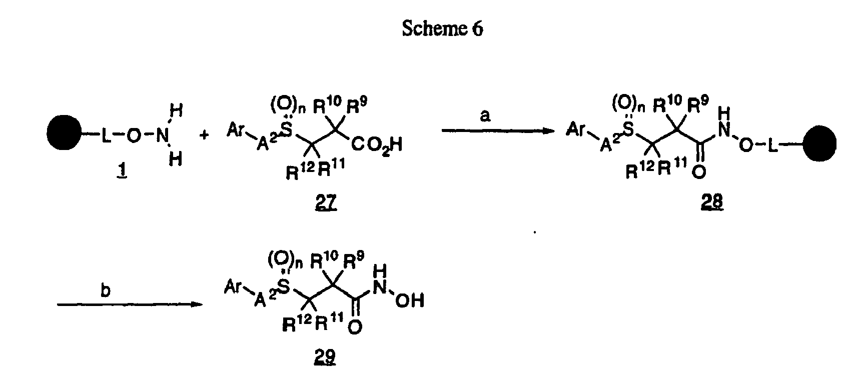

### a) 3-(4-methyoxyphenylsulfonyl) propionic acid (5 equiv); 1-(3-dimethylaminopropyl)-3-ethyl carbodiimide hydrochloride (EDCI 5 equiv.); DMF; 25°C; 12 hours. b) 50% TFA in CH₂Cl₂ (100 equiv.); 30 minutes.

According to the foregoing Scheme 6, the carboxylic acid compound 27 is coupled to the polymeric hydroxylamine resin compound 1 as described in Scheme 1 above to form the polymeric hydroxamic acid resin compound 28. The polymeric hydroxamic acid resin compound 28 is then be treated with an acid such as trifluoroacetic acid (TFA) in an inert solvent such as dichloromethane to liberate the hydroxamic acid compound 29. A higher percentage of TFA (trifluoroacetic acid) and longer reaction times are needed to cleave the hydroxamic acid from the Wang version compared to the Rink version of the resin. During the evaporation of the TFA in the work-up to isolate the hydroxamic acid, it is found that heating the sample during concentration would generate a significant amount of the N,O-diacylated dimer of the parent hydroxamic acid as a side-product. To minimize this side reaction the reaction mixture is concentrated at or below room temperature with toluene used as an azeotrope.

A process for the solid phase synthesis of the carboxylic acid compound 27 is shown in Scheme 7. In scheme 7, Ar, A², n, R⁹, R¹⁰, R¹¹ and R¹² are as hereinbefore defined.

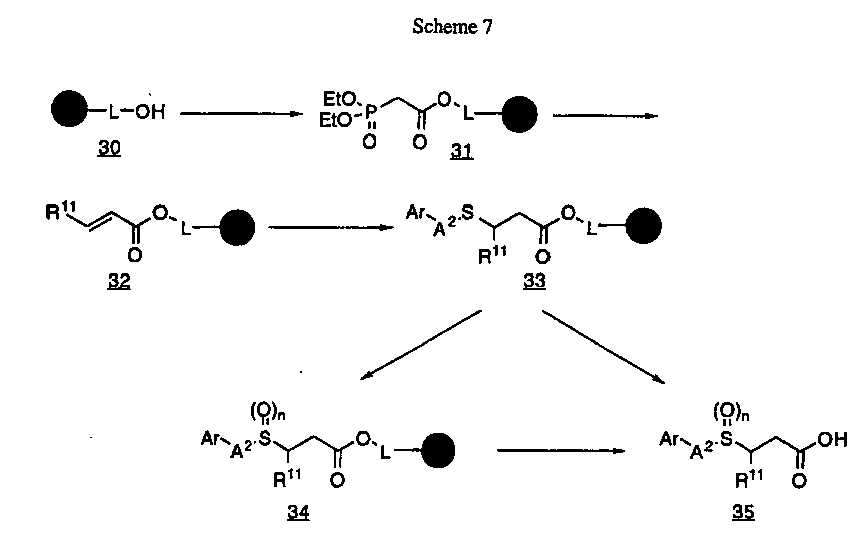

For example, the polymeric hydroxy resin compound 30 is treated with diethyl phosphonoacetic acid in an inert solvent such as dimethylformamide in the presence of 2,6-dichlorobenzoyl chloride and pyridine at a temperature at about room temperature to give the polymeric diethylphosphonoacetoxy resin compound 31.

The polymeric diethylphosphonoacetoxy-resin compound 31 is treated with a base such as potassium bis (trimethylsilyl) amide in an inert solvent such as toluene, at a temperature of about 0°C, followed by an aldehyde of formula R"CHO wherein R¹¹is as defined above, at about ambient temperature to give the polymeric alkenoate resin compound 32.

The polymeric alkenoate resin compound 32 is then reacted with a thiol of formula Ar-A²-SH wherein Ar and A² are as defined above, to give the polymeric alkanoate resin compound 33. The addition may be conveniently carried out under mild basic conditions, for example in the presence of lithium hydroxide at about ambient temperature.

The polymeric alkanoate resin compound 33 may then be hydrolyzed by treatment with an acid such as trifluomacetic acid in an inert solvent such as dichloromethane to prepare the acid 35 wherein n is 0.

Alternatively, the polymeric alkanoate resin compound 33 may be treated with an oxidizing agent such as *m*-chloro-perbenzoic acid in an inert solvent, such as dioxane at about ambient temperature to give the polymeric sulfoxide (n =1) or sulfone (n = 2) resin compound 34. Hydrolysis of 34 as described above provides the carboxylic acid compound 35.

The preparation of the polymeric hydroxylamine resin compound 1 is outlined in Scheme 8.

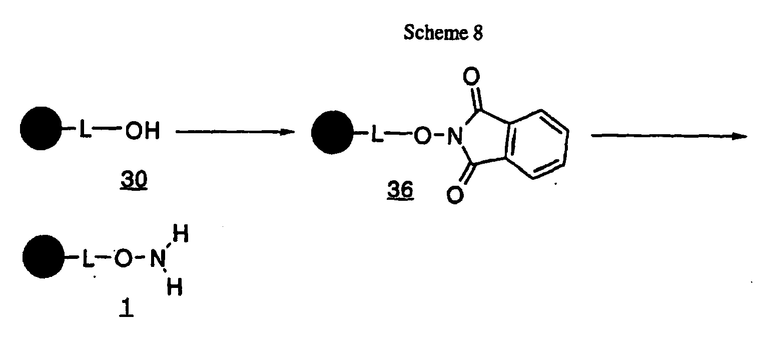

According to the foregoing Scheme 8, a polymeric hydroxy resin compound 30 is converted to the polymeric N-hydroxylphthalimidoresin compound 36 by coupling with N-hydroxyphthalimide under Mitsunobu conditions (Mitsunobu, O., *Synthesis* 1981, 1), by conversion of the hydroxy group to a leaving group such as the mesylate followed by nucleophilic diplacement, or by reaction of the polymeric hydroxy resin compound with N-hydroxyphthalimide in the presence of an acid such as benzenersulfonic acid. Removal of the phthalimido group provides the polymeric hydroxylamine resin compound 1.

For example, when 30 is 4-(hydroxymethyl)phenoxymethyl-copoly(styrene-1 %-divinylbenzene)-resin (Wang resin), N-hydroxyphthalimide is coupled to the resin in the presence of diisopropylazodicarboxylate and tripenylpbosphine in DMF. The phthalimido protection is removed by methylaminolysis in THF at 40 °C. The reaction is complete in about 2 hours. The use of the methylamine to cleave the phthalimide protection offers a significant advantage over the commonly used hydrazinolysis procedure (Wolf et al., *Can. J. Chem.,* 48, 3572, (1970).

When 4-(2',4'-dimethoxyphenyl-O-methylhydroxylamine)-phenoxymethyl-copoly(styrene-1%-divinylbenzene)-resin (Rink resin) is utilized, 1 is preferably prepared by reaction of the polymeric hydroxy resin compound with N-Hydroxy phthalimide in DMF in the presence of catalytic benzene sulfonic acid to form the polymeric N-hydroxyphthalimido resin compound 36. The phthalimido protecting group is then removed by reaction with hydrazine hydrate in tert-butanol at about 60°C to give the corresponding polymeric hydroxylamine resin compound.

An alternative route to the polymeric N-protected hydroxylamine resin 6 is outlined in Scheme 9.

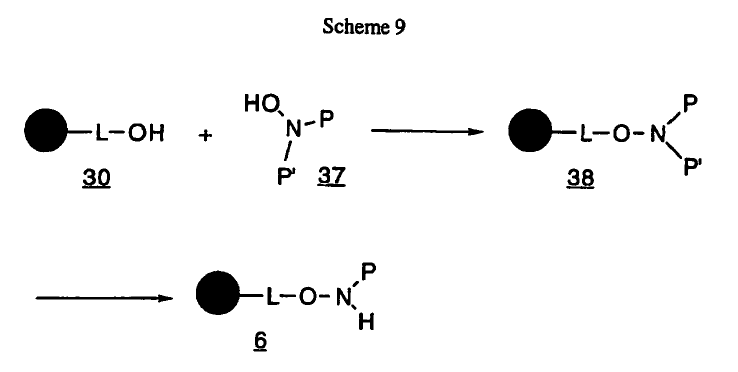

According to the foregoing Scheme 9, a polymeric hydroxy resin compound 30 is coupled with a N,N-diprotected hydroxylamine compound 37, wherein P and P' are amine protecting groups as described in Scheme 8 above to form the polymeric N,N-diprotected hydroxylamine resin compound 38. The amine protecting group P' is then selectively removed to form the polymeric N-protected hydroxylamine resin compound 6.

In a preferred embodiment of the synthesis described in Scheme 9, P is benzyl and P' is allyloxycarbonyl. Selective removal of the allyloxycarbonyl protecting group is effected by treatment with tetrakis(triphenylphosphine)Palladium(0).

The N,N-diprotected hydroxylamine compound 37 is prepared by sequential introduction of the protecting groups P and P' to an O-protected hycroxylamine compound of formula H₂NOP² wherein P² is a hydroxy protecting group. A preferred hydroxy protecting group is alkyl. The amine protecting groups P and P' are then introduced using reagents and reaction conditions well known in the art of organic synthesis. For Example, reaction of O-tert-butylhydroxylamine with allyloxychloroformate results in formation of N-allyloxycarbonyl-O-*tert*-butylhydroxylamine, which is then reacted with benzyl bromide to form N-benzyl-N-allyloxycarbonyl-O-tert-butylhydroxylamine. Treatment of N-benzyl-N-allyloxycarbonyl-O-tert-butylhydroxylanune with tritluoroacetic acid gives N-benzyl-N-allyloxycarbonylhydroxylamine.

The preparation of a polymeric 4-(O-methylhydroxylamine)-2,3,5,6-tetrafluorophenoxymethyl resin compound is shown in Scheme 10.

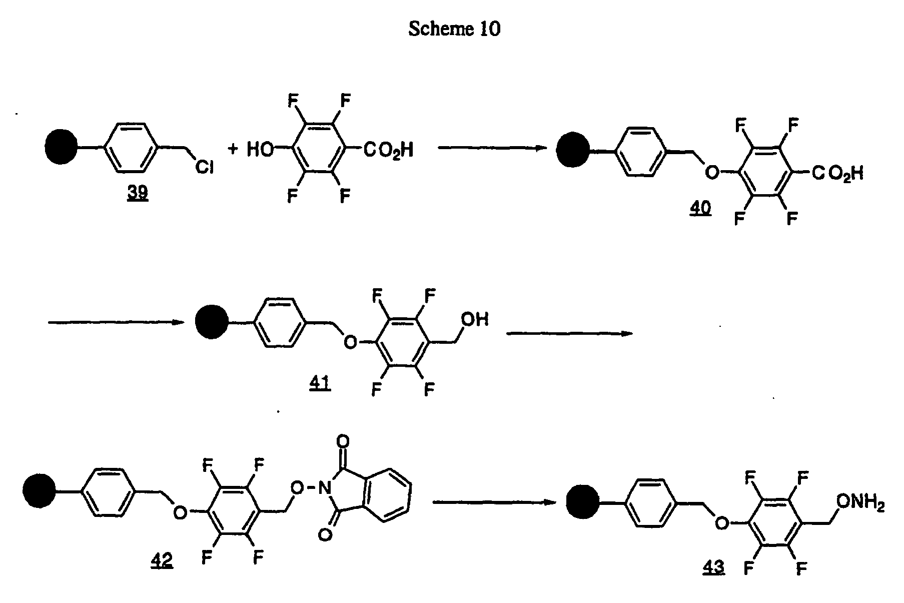

According to the foregoing Scheme 10, a polymeric chloromethyl resin compound such as chloromethylpolystyrene (39, Merrifield resin) is reacted with 4-hydroxy-2,3,5,6-benzoic acid in the presence of base to form the 4-carboxy-2,3,5,6-tetrafluorophenoxymethyl resin compound 40. Reduction of the carboxylic acid group, for example using LiAlH4, diisobutylaliuminum hydride, or BH₃-THF provides the 4-hydroxymethyl-2,3,5,6-tetrafluorophenoxymethyl resin compound 41. Conversion of 41 to the hydroxyphthalimido resin compound 42, followed by removal of the phthalimido group as described in Scheme 8 above provides the 4-(O-methylhydroxylamine)-2,3,5,6-tetrafluorophenoxymethyl-copoly(styrene-1% divinylbenzene) resin compound 43.

The preparation of a polymeric 4-(2',4'-dimethoxyphenyl-O-methylhydroxylamine)-2,3,5,6-tetrafluorophenoxymethyl resin compound is shown in Scheme 11.

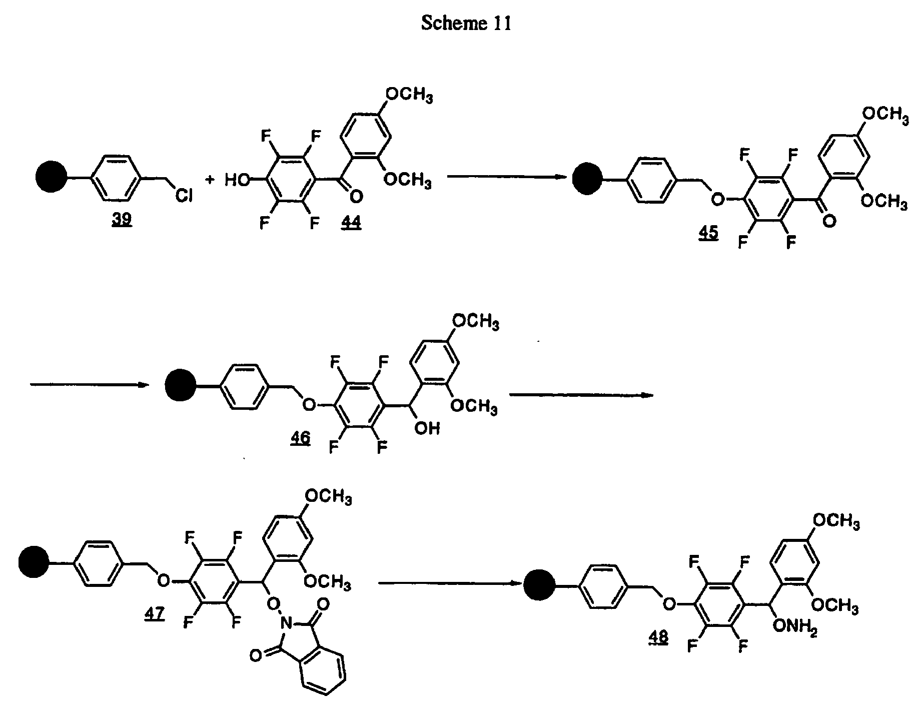

According to the foregoing Scheme 11, a polymeric chloromethyl resin compound is reacted with 4-phenoxy-2,3-5,6-tetrafluorophenyl 2,4-dimethoxyphenyl ketone 44 in the presence of base as described in Scheme 10 above to form the 4-(2',4'-dimethoxyphenylcarbonyl)-2,3,5,6-tetrafluorophenoxymethyl-resin compound 45. Reduction of the carbonyl, for example using LiBH4, provides the 4-(hydroxymethyl-2',4'-dimethoxyphenyl)-2,3,5,6-tetrafluorophenoxymethyl resin compound 46. Conversion of 46 to the hydroxyphthalimido resin compound 47, followed by removal of the phthalimido group as described in Scheme 8 above provides the 4-(2',4'-dimethoxyphenyl-O-methylhydroxylamine)-2,3,5,6-tetrafluomphenoxymethyl- resin compound 48.

Preferred polymeric hydroxylamine resin compounds have formula 1 wherein L is a linking group.

Preferred linking groups L have the formula 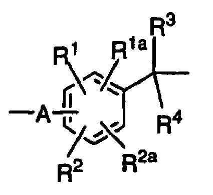 wherein
A is absent or a group of formula -X¹-Z- wherein
X¹ is -CHR- or -CHR-Y-CO-(CH₂)ₙ- wherein R is H, alkyl, phenyl, or phenyl substituted with -H, alkyl, alkoxy, halogen, nitrile or -NO₂,
Y is -O- or -NH-,
n is an integer from 1 to 6, and
Z is -O- or -NH-;
R¹, R^{1a}, R², and R^{2b} are independently -H, alkyl, alkoxy, halogen, nitrile or -NO₂; and
R³ and R⁴ are independently -H, alkyl, phenyl, or phenyl substituted with one or more substituents selected from alkyl, alkoxy, halogen nitrile and -NO₂;
or one of R¹ and R² taken together with one of R³ and R⁴ and the carbon atoms to which they are attached define a linking group of formula 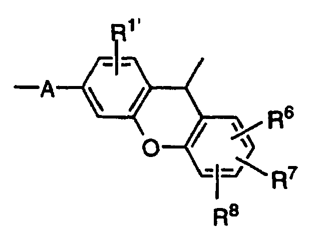 wherein
R¹ is -H, alkyl, alkoxy, halogen, nitrile or -NO₂; and
R⁶, R⁷ and R⁸ are independently selected from -H, alkyl, allcoxy, halogen, nitrile or -NO₂.

Representative preferred polymeric hydroxylamine resin compounds include 4-(O-methylhydroxylamine)phenoxymethyl-copoly(styrene- 1% divinylbenzene) resin, designated herein as 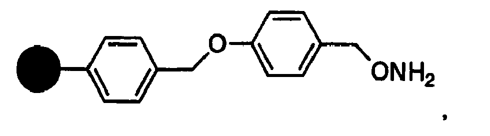 4-[4-(O-methylhydroxylamine)-3-methoxyphenoxy]-(N-4-methylbenzhydryl)-butyramide-copoly(styrene-1 %-divinylbenzene)-resin, designated herein as 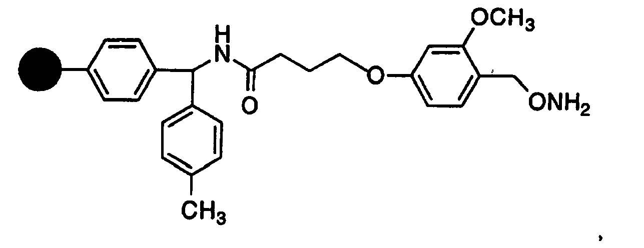 4-(2',4'-dimethoxyphenyl-O-methylhydroxylamine)-phenoxymethyl-copoly(styrene-1%divinylbenzene) resin, designated herein as 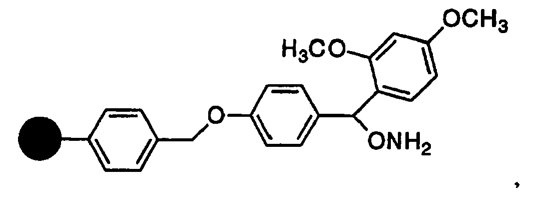 4-[4-(1-aminoxyethyl)-2-methoxy-5-nitrophenoxy]-(N-4-methylbenzhydryl)-butyramide-copoly(styrene-1% divinylbenzene) resin, designated herein as 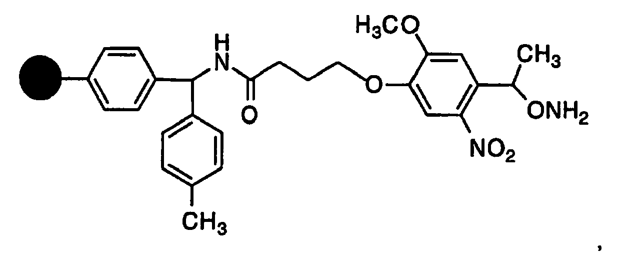 O-hydroxylamine-2' -chlorotrityl-copolystyrene-1%-divinylbenzene-resin, designated herein as 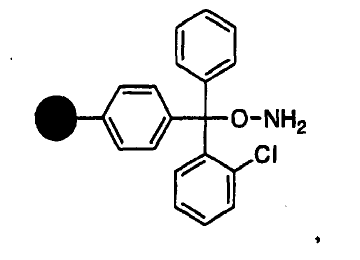 O-hydroxylamine-trityl-copolystyrene-1 %-divinylbenzene-resin, designated herein as 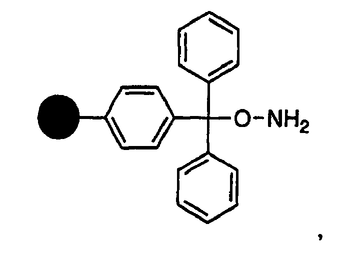 5-(4-O-methylhydroxylamine -3,5-dimethoxyphenoxy)-valeric acid-copolystyrene-1 %-divinyl benzene resin, designated herein as 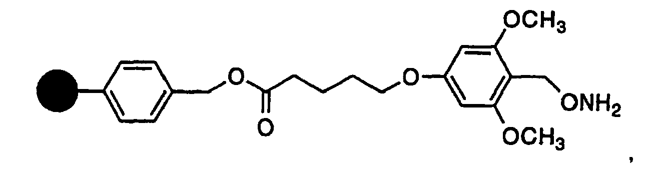 4-O-methylhydroxylamine-3-methoxyphenoxy-copolystyrene-1%-divinyl benzene resin, designated herein as 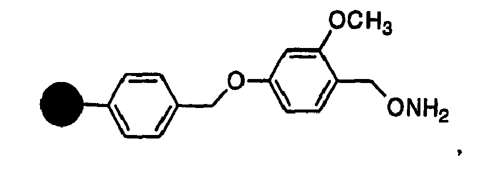 3-hydroxy-xanthydroxylamine-copolystryene-1%-divinylbenzene resin, designated herein as 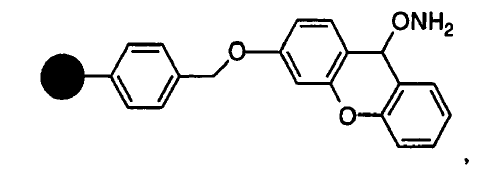 4-(O-methylhydroxylamine)-2,3,5,6-tetrafluorophenoxymethyl-copoly(styrene-1% divinylbenzene) resin, designated herein as 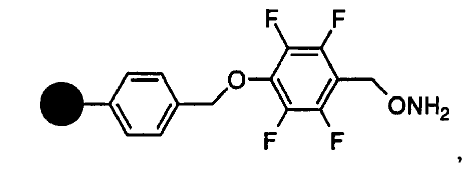 and 4-(2',4'-dimethoxyphenyl-O-methylhydroxylamine)-2,3,5,6-tetrafluorophenoxymethyl-copoly(styrene-1 % divinylbenzene) resin, designated herein as

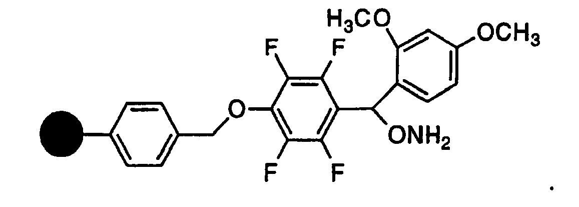

The most preferred polymeric hydroxylamine resin compounds are 4-(O-methylhydroxylamine)-2,3,5,6-tetrafluorophenoxymethyl-copoly(styrene-1% divinylbenzene) resin, 4-(2',4'-dimethoxyphenyl-O-methylhydroxylamine)-2,3,5,6-tetrafluorophenoxymethyl-copoly(styrene-1% divinylbenzene) resin, 4-(O-methylhydroxylamine)phenoxymethyl-copoly(styrene-1% divinylbenzene) resin, and 4-(2',4'-dimethoxyphenyl-O-methylhydroxylamine)-phenoxymethyl-copoly(styrene-1% divinylbenzene) resin.

The Rink handle (H. Rink, *Tet. Lett.,* 28, 3787-3790,1987) has the advantage of being cleaved under mild acidolysis for short periods of time (i.e. 10% TFA in DCM for 10-15 minutes.). However, due to the cost of the resin it is desirable to synthesize the corresponding functional resin on the Wang solid support ((a) S. S. Wang, *J. Am. Chem. Soc.,* (1973), 95, 1328. b) G. Lu, S. Mojsov, J. P. Tam, and R. B. Merrifield, *J. Org. Chem.,* (1981), 46, 3433).

The tetrafluorophenylphenyl handle is especially useful as it lends itself to ready quantification of resin loading and monitoring of reactions conducted on the resin using fluorine NMR.

The methods described herein are also useful for the preparation of peptide aldehydes, ketones and hydroxamic acids. In general, this method involves coupling the carboxyl group of a suitably N-protected first amino acid to the resin to form the polymeric N-protected amino acid hydroxamic acid resin compound. The amino acid N-protecting group is then removed and the unprotected polymeric amino acid hydroxamic acid resin compound is coupled with a second suitably N-protected amino acid. This process is then repeated until the desired amino acid residues have been incorporated in the peptide.

Alternatively, peptides comprising multiple amino acids are prepared by coupling a suitably N-protected peptide subunit comprising two or more amino acids to form the polymeric N-protected peptide hydroxamic acid resin compound. The amino acid N-protecting group is then removed and the unprotected polymeric peptide hydroxamic acid resin compound is coupled with a second suitably N-protected amino acid or peptide. Thus, in addition to the sequential addition of individual amino acid subunits described above, a polypeptide may be prepared by coupling of peptide subunits.

Once the desired amino acids have been incorporated into the peptide, the polymeric peptide hydroxamic acid compound is reacted with ah organometallic reagent followed by acid hydrolysis to form the peptide ketone compound; reductively cleaved to form the peptide aldehyde compound; or cleaved with acid to form the peptide hydroxamic acid compound. Any remaining protecting groups may be removed prior to or subsequently to cleavage of the peptide from the resin.

N-protecting groups suitable for use in peptide synthesis as described herein should have the properties of being stable to the conditions of coupling to the polymeric hydroxylamine resin compound while being readily removable without destruction of the growing peptide chain or racemization of any of the chiral centers contained therein. Suitable protecting groups are 9-fluorenylmethyloxycarbonyl (Fmoc), *t*-butyloxycarbonyl (Boc), benzyloxycarbonyl (Cbz), biphenylisopropyloxycarbonyl, *t-*amyloxycarbonyl, isobornyloxycarbonyl, (a,a)dimethyl-3,5-dimethoxybenzyloxycarbonyl, o-nitrophenylsulfenyl, 2-cyano-*t*-butyloxycarbonyl, and the like.

Additionally, this resin is useful for constructing arrays of aldehyde, ketone or amine combinatorial libraries or arrays of aldehydes and ketones as reagents in combinatorial library synthesis, for example reagents for the Ugi 4-component condensation (Ivar Ugi, in Isonitrile Chemistry, 1971, p. 145, Academic Press). The hydroxylamine bound resin may be used not only for single functional group transformations, but also multiple step solid phase synthesis to generate combinatorial libraries.

The functionalized resin of this invention is also useful for the parallel synthesis of a multiplicity of different aldehyde, ketone or amine end products as outlined for ketone compounds in Schemes 12a and 12b. In Schemes 12a and 12b, R_{b} and R_{c} are as defined above. n is an integer which represents the total number of different aldehyde, ketone or amine products which are to be prepared. Rₐₗ-Rₐₙ represent, independently, an aliphatic or aromatic group as defined herein.

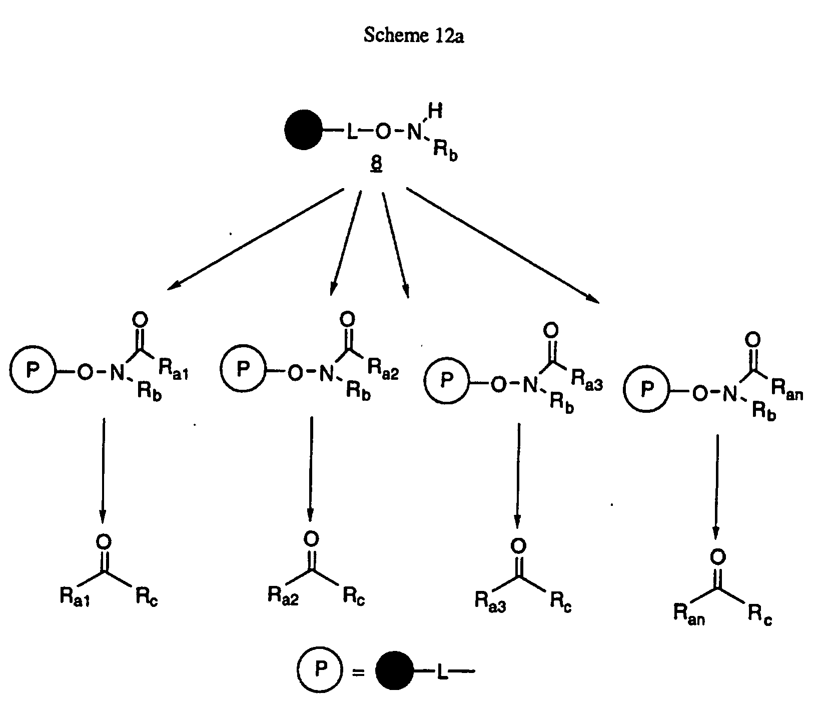

The parallel synthesis of a multiplicity of ketone compounds using a multiplicity of carboxylic acid compound RₐₗCO₂H-RₐₙCO₂H and a single organometallic compounds R_{c}MgX is shown in Scheme 12a. According to Scheme 12a, the N-alkylated hydroxylamine resin compound 8, prepared as described in Scheme 2, is divided into n portions. Each portion of resin is then coupled with a different carboxylic acid compound to give n portions of polymeric N-alkylated hydroxamic acid resin compound. Each portion of polymeric N-alkylated hydroxamic acid resin compound is then reacted with a Grignard reagent of formula R_{c}X and subjected to acid hydrolysis to give n portions of ketone derived from a single organometallic reagent.

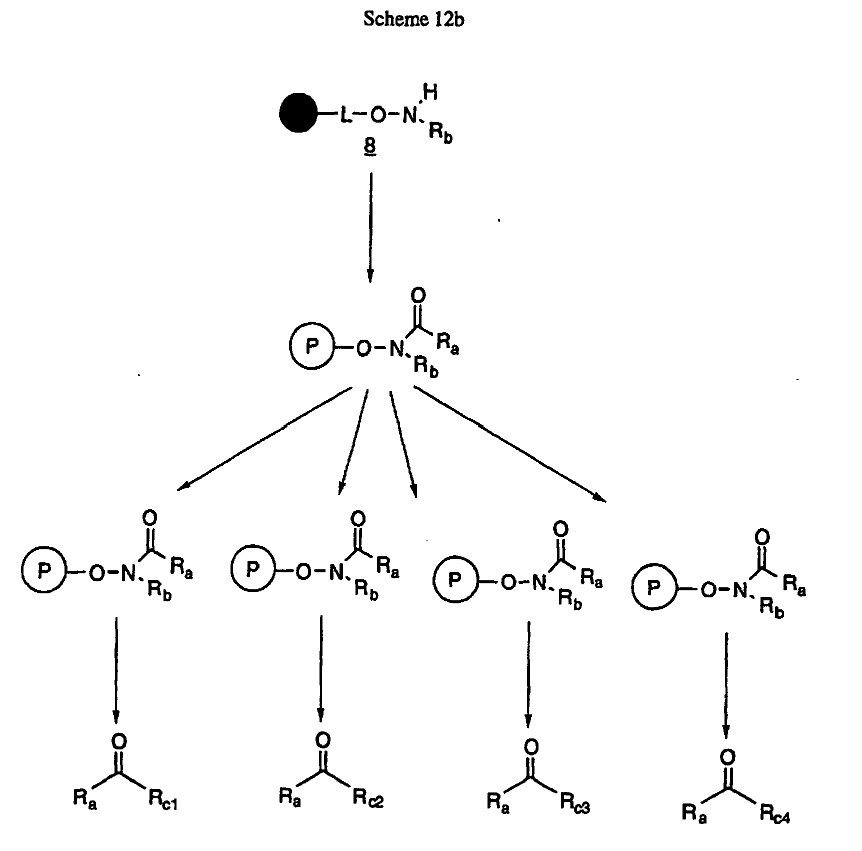

The parallel synthesis of n different ketone compounds derived from a single carboxylic acid compound RₐCO₂H and n different organometallic compounds R_{c1}MgBr to R_{ca}MgBr is outlined in Scheme 12b above. According to Scheme 12b, the polymeric N-alkylated hydroxylamine resin compound is coupled with a carboxylic acid of formula RₐCO₂H. The resulting polymeric N-alkylated hydroxamic acid resin compound is then divided into n portions, and each portion of polymeric N-alkylated hydroxamic acid resin compound is then reacted with a different Grignard reagent R_{c1}-R_{cn}MgBr and subjected to acid hydrolysis to give n different ketone compounds derived from a single carboxylic acid compound.

The functionalized resins of this invention are also useful for constructing a combinatorial library of ketones or amines as illustrated for the ketone library derived from 4 carboxylic acid compounds and 4 Grignard reagents as outlined in Scheme 13.

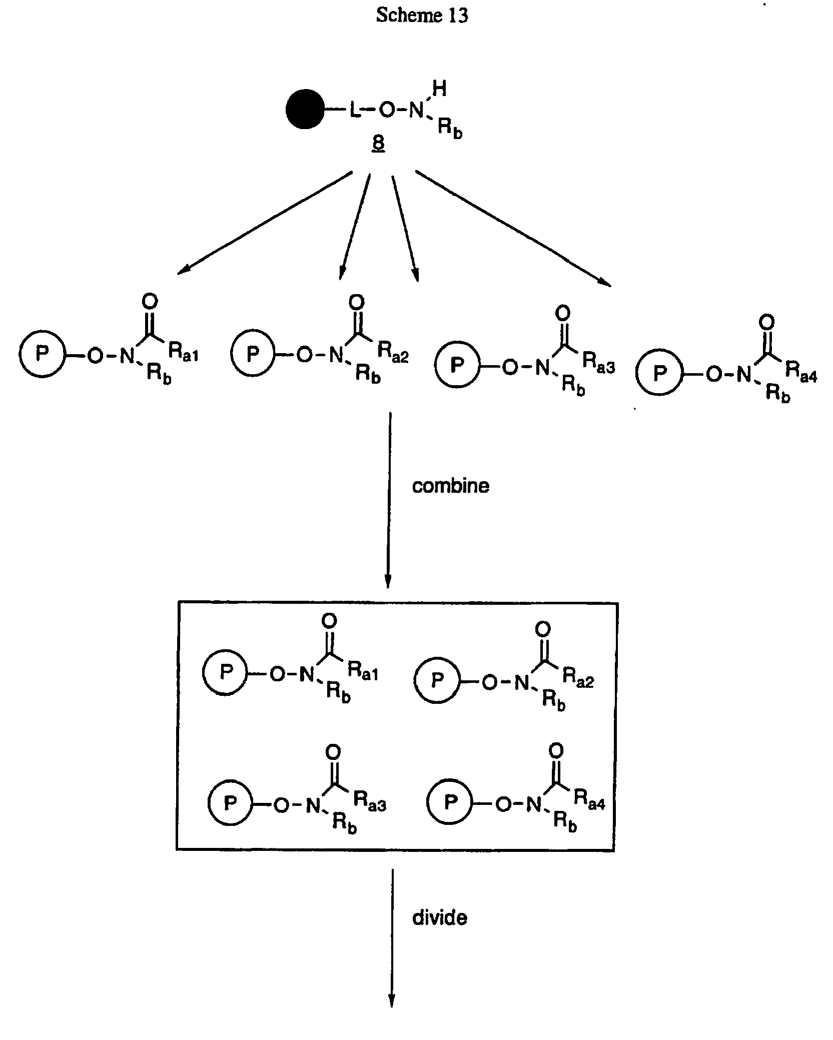

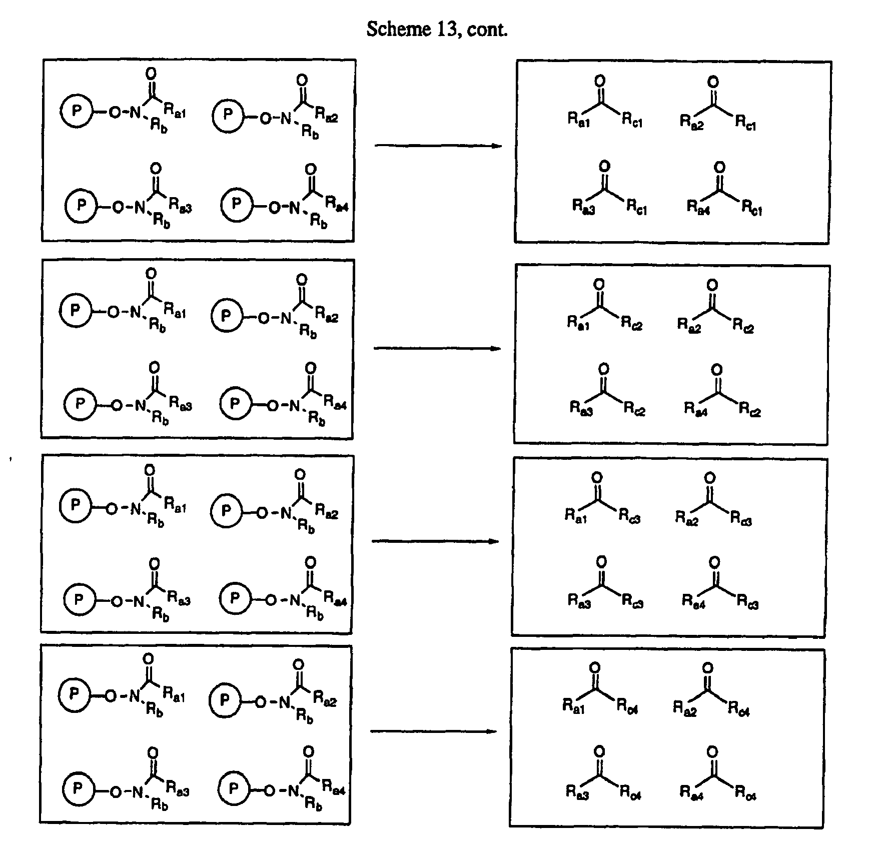

According to the foregoing Scheme 13, the polymeric N-alkylated hydroxylamine resin compound 8 is divided in 4-portions, and each portion is coupled with a different carboxylic acid compound to prepare 4 different polymeric N-alkylated hydroxamic acid resin compounds. The 4 portions of polymeric N-alkylated hydroxamic acid resin compounds are then mixed together to form a single portion which is then divided into 4 portions of polymeric N-alkylated hydroxamic acid resin compounds, in which each portion contains approximately equal amounts of each individual polymeric N-alkylated hydroxamic acid resin compound. Each of the 4 portions is then reacted with a different Grignard reagent R_{c1}-R_{c4}MgBr and subjected to acid hydrolysis to give 4 portions of ketone compound, each of which contains 4 compounds representing the products of reaction of each of the 4 different polymeric N-alkylated hydroxamic acid resin compounds with a single Grignard reagent. In this manner a combinatorial library containing a multiplicity of ketone compounds may be quickly constructed.

In a similar manner, a combinatorial library of peptides may be assembled by repeating the dividing-recombining sequence for each amino acid or peptide building block.

The foregoing may be better understood by reference to the following Examples, which are presented for illustration and not intended to limit the scope of the invention.

### Example 1

### Preparation of 4-(2' ,4' -dimethoxyphenyl-O-methylhydroxylamine)-phenoxymethyl-copoly(styrene-1% divinylbenzene) resin.

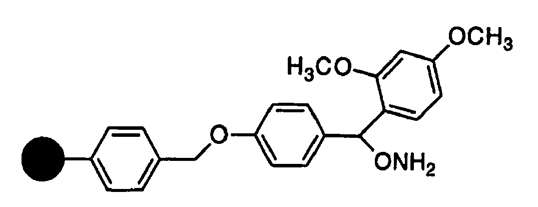

Rink acid resin (1 g; 0.63 mmol) is swelled in DMF (10 mL) for 1 minutes at ambient temperature. N-Hydroxyphthalimide (514 mg; 3.15 mmol) is added to the resin suspension followed by benzene sulfonic acid (19 mg; 0.13 mmol). The mixture is stirred by means of a mechanical stirrer and heated to 50°C for five hours. The mixture is then cooled to ambient temperature and stirred for an additional 12 hours, after which the resin is filtered and washed extensively with DMF (5 x 25 mL); DMF:H₂O (70:30; 5 x 25 mL); THF (10 x 25 mL); and diethyl ether (10 x 25 mL). The resin is then dried overnight under high vacuum at 40 °C. The IR spectrum shows a carbonyl absorbance at 1733 cm⁻¹ corresponding to the phthalimido carbonyl stretch. Elemental analysis: calcd.:0.28% N. Found: 0.26%N. Loading = 0.18 mmol/g.

The resin is swelled in 20 mL of tert-butanol for ten minutes. Hydrazine hydrate (10 mL) is added to the mixture and the reaction is warmed to 60 °C with mechanical stirring for 12 hours. After which the reaction is cooled to ambient temperature. The resin is filtered and washed extensively with DMF (10 x 25 mL), THF (10 x 25 mL), and diethyl ether (10 x 25 mL), then dried under high vacuum at 40 °C overnight. The IR spectrum of resin III showed the loss of the carbonyl stretch at 1733 cm⁻¹ which is present in the starting material. Elemental Analysis: %N found = 0.43; 0.42 (coresponding to a loading level of 0.3 mmol/g).

### Example 2

### Preparation of N-[3-((4-methoxyphenyl)sulfonyl)prop-1-ylcarbonyl]4-(2',4'-dimethoxyphenyl-O-methylhydroxylamine)-phenoxymethyl-copoly(styrene-1% divinylbenzene) resin.

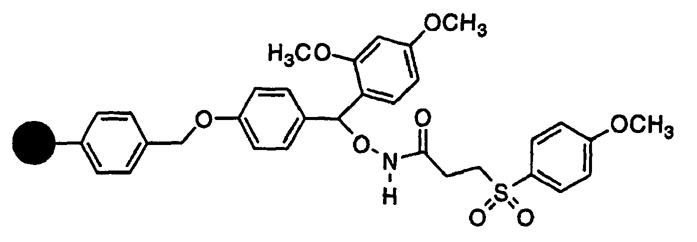

4-(2',4'-dimethoxyphenyl-O-methylhydroxylamine)-phenoxymethyl-copoly(styrene-1% divinylbenzene) resin (200 mg) is swelled in DMF (3 mL). To this suspension is added 3-(4-methoxyphenylsulfonyl)propionic acid (610 mg; 2.5 mmol) and 1-(3-dimethylaminopropyl)-3-ethyl carbodiimide hydrochloride (EDCI; 477 mg; 2.5 mmol) at ambient temperature. The reaction mixture is shaken at ambient temperature using a vortex shaker for 12 h, after which the resin is filtered and washed extensively with DMF:H₂O (80:20; 5 x 5 mL.), DMF (5 x 5 mL), THF (5 x 5 mL), and diethyl ether (5 x 5 mL). The resin IV is dried under high vacuum at 40 °C for 12 hours. The IR spectrum shows a carbonyl absorbance at 1675 cm⁻¹ corresponding to the bound hydroxamate.

### Example 3

### Preparation of N-hydroxy-3-(4-methoxyphenylsulfonyl)propionamide.

Dry N-[3-((4-methoxyphenyl)sulfonyl)prop- -ylcarbonyl]4-(2',4'-dimethoxyphenyl-O-methylhydroxylamine)-phenoxymethyl-copoly(styrene-1% divinylbenzene) resin (200 mg), prepared as in Example 2, is swelled in 3 mL of methylene chloride for 10 minutes. Trifluoroacetic acid (TFA; 0.3 mL) is added to the mixture dropwise at ambient temperature and the resulting mixture is vortexed for 30 minutes. The resin turned a dark blue upon addition of the TFA. The mixture is then filtered and washed with two 5 mL portions of methylene chloride. The filtrate is evaporated by rotary evaporation to yield 20 mg of crude product. An LC/MS trace of the crude reaction mixture showed it to contain better than 75 area % of the desired product, 3-(4-methoxyphenylsulfonyl)propionic acid is present in 6 area %). ¹H NMR (MeOH-d₄) δ 2.45 (t,2H); 3.45 (t,2H); 3.90 (s,3H), 7.15 (d, 2H); 7.85 (d, 2H).

### Example 4

### Preparation of 4-O-Methylhydroxylamine)phenoxymethyl-copoly(styrene-1%-divinylbenzene)-resin (100-200 mesh).

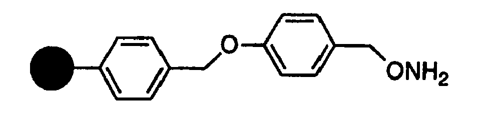

A 1-L jacketed reactor with a bottom valve and overhead stirrer (Ace catalog #8090) is charged with Wang resin (18.35 g, 20 meq) and anhydrous tetrahydrofuran (THF, 450 mL). This mixture is stirred gently for about 15 minutes, then as much solvent as possible is removed through a tube fitted with a porous glass frit via vacuum aspiration. Fresh THF is added, followed by triphenylphosphine (15.74 g, 60 mmol) and N-Hydroxyphthalimide (16.31 g, 100 mmol). The resulting mixture is stirred and cooled to -5-0 °C. Diisopropyl azodicarboxylate (11.8 mL, 60 mmol) is added slowly so as to maintain the temperature at <5 °C. When the addition is complete, the stirred mixture is allowed to warm slowly to room temperature and stirred overnight. As much of the reaction liquors as possible is removed by aspiration through the dip tube as above. The resin is washed by charging N,N-dimethylformamide (DMF, 200 mL), stirring the mixture for 3-5 minutes, and then removing by aspiration as much of the wash solution as possible. Similarly, the resin is washed sequentially with an additional portion of DMF and portions of methanol (twice), THF (twice), and methanol (once). A portion of the resin may be removed for analysis: IR 1734 cm-1 (C=O).

To the resin remaining in the reactor is added THF (400 mL) and 200 mL of a 40% aqueous solution of methylamine (2.31 mol). This reaction mixture is stirred gently at 40 °C for 2 hours, then cooled to room temperature (the mixture may be held overnight at this temperature). As much of the reaction liquor as possible is removed by aspiration, and the resin is washed with the solvent array as above. Following the final methanol wash, additional methanol is used to flush the resin out of the bottom of the reactor and isolate it by filtration. The filtered resin is dried at 40 °C under vacuum. Yield 18-18.5 g resin: amine load 1.02 meq/g (based on potentiometric titration of a THF suspension with p-toluenesulfonic acid); IR (microscopy) 3316 cm⁻¹ (w, -NH₂). Analysis found C, 87.07%; H, 7.77%; N, 1.58%, which corresponds to 1.13 nitrogen atoms/g resin.

### Assay: preparation of 4-nitrophenylethanehydroxamic acid.

A 200 mg sample of the dried resin (ca. 0.2 mmol) is charged to a 5- or 10-mL resin reactor (a polypropylene syringe barrel fitted with a polypropylene frit). The resin is swelled for about 15 minutes in dry DMF, and then 115 mg 1-(3-dimethylaminopropyl)-3-ethylcarbodiimide hydrochloride (EDCI, 0.6 mmol) is added. To this mixture is then added 4-nitrophenylacetic acid (115 mg, 0.6 mmol). The reactor is capped and the mixture is agitated slowly overnight (a rocker bed apparatus is used). The reaction liquors are removed by vacuum filtration (the resin reactor is inserted through a small rubber vacuum flask adapter), and the resin is washed by several small (2-3 mL) portions of the following solvents: DMF (4-5 portions), MeOH or 50% aq. DMF (3-4 portions), THF (3-4 portions), and MeOH (2-3 portions). The resin (still in the syringe reactor) is dried for at least 4 hours under vacuum at 40 °C.

To this dried resin is added 2 mL dichloromethane followed by 2 mL trifluoroacetic acid (TFA). Additionally, 20 mL water is added (believed to reduce "anhydride" formation from hydroxamic acid product). The mixture is allowed to react for about 1 hr, and the reaction liquors are drained into a tared collector. The resin is washed with 1-2 1-mL portions of dichloromethane followed by 1-2 1-mL portions of toluene. The combined filtrates are concentrated to about 2 mL at 30°C, 2 mL additional toluene is added, and the resulting solution is concentrated to dryness under vacuum (rotary evaporator followed by vacuum oven at 30 °C; note that heating in the presence of TFA promotes formation of the "anhydride" impurity). The residue is weighed and analyzed for weight % purity (HPLC, using the carboxylic acid as a response factor standard). Typical results for 4-nitrophenylethanehydroxamic acid: 29-30 mg solids at 60-70 wt% purity, 90-97 A% purity (261 nm); ¹H NMR (CD₃OD) 8 8.13 (d, 2H), 7.25 (d, 2H), 4.85 (bs, OH, NH), 3.55 (s, 2H); ¹³C NMR δ 169.4, 144.3, 131.3, 124.6, 40.2. This reflects a load/clip chemical yield of 50-55% from resin at 1 meq/g.

### Example 5

### Preparation of N-4-phenylbut-1-oyl-4-0-methylhydroxylamine)phenoxymethyl-copoly(styrene-1%-divinylbenzene)-resin.

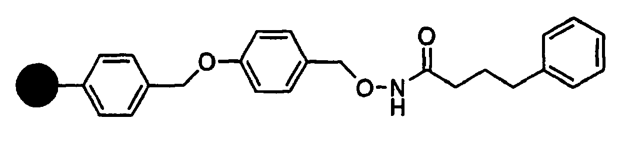

Dry 4-O-Methylhydroxylamine)phenoxymethyl-copoly(styrene-1%-divinylbenzene)-resin (2 g, 1.5 mmol), prepared as in Example 4, is allowed to swell in DMF (8 mL) for 10 minutes and then is treated with 4-phenyl butyric acid and EDC (0.86 g, 4.5 mmol). The mixture is shaken for 24 hours and filtered. The resin is washed with DMF, DMF/H₂O, DMF, THF and Et₂O and dried under vacuum at 40°C to give N-4-phenylbut-1-oyl- 4-O-methylhydroxylamine)phenoxymethyl-copoly(styrene-1%-divinylbenzene)-resin (2.2 g). IR: C=O 1670 cm⁻¹. Elemental analysis: calcd; N, 1.05%. Found: N, 1.07%.

### Example 6

### Preparation of N-(4-bromo-3-methylbenzoyl)-4-O-methylhydroxylamine)phenoxymethyl-copoly(styrene-1%-divinylbenzene)-resin.

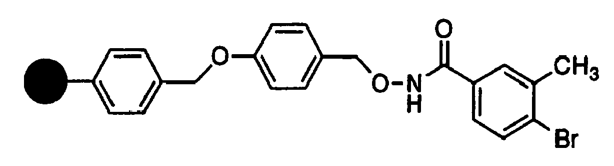

Dry 4-O-Methylhydroxylamine)phenoxymethyl-copoly(styrene-1%-divinylbenzene)-resin (4 g, 3 mmol), prepared as in Example 4, is allowed to swell in DMF (32 mL) for 10 minutes, then is treated with 4-bromo-3-methylbenzoic acid and EDC (1.725g, 9 mmol). The mixture is shaken for 24 hours and filtered. The resin is washed with DMF, DMF/H₂O. DMF, THF and Et₂O and dried under vacuum at 40°C to give N-(4-bromo-3-methylbenzoyl)-4-O-methylhydroxylamine)phenoxymethyl-copoly(styrene-1%-divinylbenzene)-resin (4.5 g). IR: C=O 1677.5cm⁻¹. Elemental analysis: calcd: Br, 5.2%; N, 1.05%. Found: Br, 5.3%; N, 0.91%.

### Example 7

### Preparation of N-Hydroxy-4-bromo-3-methyl benzamide.

N-(4-bromo-3-methylbenzoyl)-4-O-methylhydroxylamine)phenoxymethyl-copoly(styrene-1%-divinylbenzene)-resin, prepared as in Example 6, is suspended in 50% TFA/CH₂Cl₂ for 2 hours. The resin is filtered and washed three times with CH₂Cl₂ to give N-Hydroxy-4-bromo-3-methyl benzamide. LC MS: m/z 230/232 (Br)[M+H]⁺ Area = 78%; ¹H NMR (300 Mhz, CDCl₇,) δ: 2.42 (s, 3H), 7.4 (bd J = 7.89,1 1H) 7.58 (bd J = 7.891 1H), 7.62 (bs,1 1H).

### Example 8

### Preparation of N-4-bmmobenzyl-N-4-phenylbut-1-ylcarbonyl- 4-O-methylhydroxylamine)phenoxymethyl-copoly(styrene-1%-divinylbenzene)-resin.

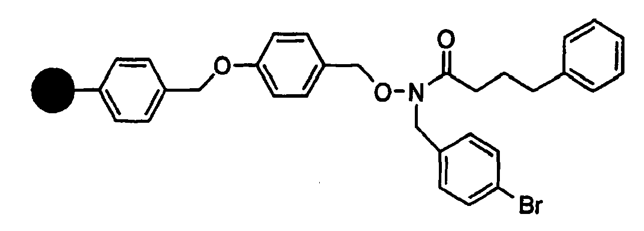

N-4-phenylbut-1-oyl-4-O-methylhydroxylamine)phenoxymethyl-copoly(styrene-1%-divinylbenzeney-resin (1.46 g, 1.095 mmol), prepared as in Example 5, is suspended in toluene (26 mL) for 10 minutes. DBU (0.83 mL; 5.5 mmol) is added and the mixture is agitated for 2 hours on a wrist shaker. Bromobenzyl bromide (4.1 g, 16.425 mmol) is added and the reaction mixture is vigorously agitated for 4 days. The resin is filtered and washed with DMF, DNF/H₂O, DMF, THF and Et₂O and dried under vacuum at 40°C to give N-4-bromobenzyl-N-4-phenylbut-1-ylcarbonyl-4-O-methylhydroxylamine)phenoxymethyl-copoly(styrene-1%-divinylbenzene)-resin (1.4 g). IR C=O 1668 cm⁻¹. Elemental Analysis: calcd: Br, 5.3%; N,0.94%. Found: Br, 5.4%; N, 0.85%.

### Example 9

### Preparation of 4-Phenyl butyraldehyde.

N-4-bromobenzyl-N-4-phenylbut-1-oyl-4-O-methylhydroxylamine)phenoxymethyl-copoly(styrene-1 %-divinylbenzene)-resin (0.2 g, 0.6 mmol/g 0.12 mmol) is suspended in diethyl ether for 10 minutes and then cooled to 5 °C in an orbital shaker. The suspension is treated with LiAlH₃OMe (0.46 M in diethyl ether, 0.22 mL, 0.1 mmol) and agitated for 30 minutes at this temperature. The reaction mixture is quenched by the addition of 2 M HCL (aq) and vortexed for 30 minutes. Sodium potassium tartrate is added and the mixture vortexed for a further 10 minutes. Sodium sulfate is added and the mixture is filtered through a plug of silica gel, washing thoroughly with dichloromethane. The filtrate is concentrated to give 4-phenyl butyraldehyde. GC: Area = 91 %; ¹H NMR (CDCl₃) δ 9.75 (1H,s), 7.05-7.30 (5H,m), 2,58-2.68 (2H,m), 2,41-2,50 (2H,t), 1,91-2.02 (2H,m); MS (EI): m/z = 149 [M+H⁺].

### Example 10

### Preparation of 6-Phenylhexan-3-one.

N-4-bromobenzyl-N-4-phenylbut-1-oyl-4-O-methylhydroxylamine)phenoxymethyl-copoly(styrene-1 %-divinylbenzene)-resin (0.15 g, approx. 0.75 mmol/g 0.11 mmol) is suspended in diethyl ether (1 mL) and treated with 1 M solution of ethyl magnesium bromide in tetrahydrofuran (0.34 mL, 0.34 mmol). The reaction mixture is agitated for 18 hours, and then quenched by the addition of 2 M HCl (aq) (approx pH 3 is obtained). The mixture is agitated for 30 minutes. Sodium sulfate is added and the mixture is filtered through a plug of silica gel, washed thoroughly with dichloromethane and concentrated to give of 6-phenylhexan-3-one. GC MS (EI) Area = 97.1%, m/z 176.2 (M)⁺; MS (EI-LRP) m/z 176 (M)⁺; NMR (300 Mhz, CDCl₃) δ 1.02 (t, 3H), 1.9 (m, 2H), 2.4 (m, 4H) 2.6 (m, 2H), 7.2-7.3 (m, 5H).

### Example 11

### Preparation of N-4-chlorobenzyl-N-(4-bromo-3-methylbenzoyl)-4-O-methylhydroxylamine)phenoxymethyl-copoly(styrene-1%-divinylbenzene}-resin.

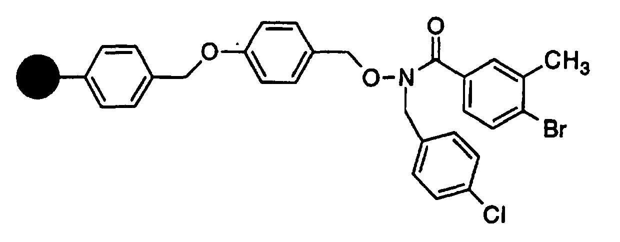

N-(4-bromo-3-methylbenzoyl)-4-O-methylhydroxylamine)phenoxymethyl-copoly(styrene-1%-divinylbenzene)-resin (2.8 g, 2.1 mmol), prepared as in Example 6, is suspended in toluene (27 mL) and the mixture is stirred for 10 minutes. DBU (1.6 g, 10.5 mmol) is added and the mixture is agitated for 2 hours on a wrist shaker. Chlorobenzyl bromide (6.47 g, 31.5 mmol) is added and the reaction mixture is vigorously agitated for 3 days. The resin is filtered and washed with DMF, DMF/H₂O, DMF, THF and Et₂O, and dried under vacuum at 40°C to give N-4-chlorobenzyl-N-(4-bromo-3-methylbenzoyl)-4-O-methylhydroxylamine)phenoxymethyl-copoly(styrene-1 %-divinylbenzene)-resin (3 g). IR C=O 1644 cm⁻¹; Elemental Analysis: calcd: Br, 4.2%; Cl, 1.9%; N, 0.8%. Found: Br, 3.8%; Cl, 2.0%; N, 0.9%.

### Example 12

### Preparation of N-(4-Chlorobenzyl)-N-hydroxy-3-methyl-4-bromobenzamide.

N-4-chlorobenzyl-N-(4-bromo-3-methylbenzoyl)-4-O-methylhydmxylamine)phenoxymethyl-copoly(styrene-1%-divinylbenzene)-resin, prepared as in Example 11, is suspended in 50% TFA/CH₂Cl₂ for 2 hours. The resin is filtered and washed three times with CH₂Cl₂ to give N-(4-Chlorobenzyl)-N-hydroxy-3-methyl-4-bromobenzamide. LC MS (H-ISP) m/z 354/356 (Cl/Br) [M+H]⁺ Area 64%;¹H NMR (300 Mhz, CDCl₃) δ 2.3 (bs, 3H), 4.65 (bs, 2H), 7.2-7.6 (m, 7H).

### Example 13

### Preparation of 4-Bromo-3-methyl benzaldehyde.

N-4-chlorobenzyl-N-(4-bromo-3-methylbenzoyl)-4-O-methylhydroxylamine)phenoxymethyl-copoly(styrene-1 %-divinylbenzene)-resin (0.2 g, 0.5 mmol/g 0.1 mmol), prepared as in Example 11, is suspended in diethyl ether for 10 minutes and then cooled to 5 °C in an orbital shaker. The suspension is treated with LiAlH₃OMe (0.46 M in diethyl ether, 0.2 mL, 0.092 mmol) and agitated for 30 minutes at this temperature. The reaction mixture is quenched by the addition of aqueous 2 M HCL and vortexed for 30 minutes. Sodium potassium tartrate is added and the mixture is vortexed for a further 10 minutes. Sodium sulfate is added and the mixture is filtered through a plug of silica gel, washing thoroughly with dichloromethane. The filtrate is concentrated to give 4-bromo-3-methyl benzaldehyde: GC MS: EI Area = 99.5%, m/z 179/199 (Br)[M]⁺;¹H NMR (CDCl₃) δ 9.94 (1H,s), 7.70 (2H,d), 7.52 (1H,d), 2.45 (3H,s); MS (EI): m/z=199 [M+H⁺].

### Example 14

### Preparation of 1-(4-Bromo-3-methyl phenyl) propan-1-one.

N-4-chlorobenzyl-N-(4-bromo-3-methylbenzoyl)-4-O-methylhydroxylamine)phenoxymethyl-copoly(styrene-1%-divinylbenzene)-resin (0.23 g, 0.5 mmol/g 0.115 mmol), prepared as in Example 11, is suspended in diethyl ether (1 mL) and treated with ethyl magnesium bromide (1.0 M in THF, 0.23 mL, 0.23 mmol). The reaction mixture is agitated for 18 hours, and then quenched by the addition of aqueous 2 M HCL (approx pH 3 is obtained). The mixture is agitated for 30 minutes. Sodium sulfate is added and the mixture is filtered through a plug of silica gel, washing thoroughly with dichloromethane. The residue is concentrated to give1-(4-Bromo-3-methyl phenyl) propan-1-one. GC Area = 78.7%; MS (EI) m/z 226 Br [M⁺-H]; NMR (300 Mhz, CDCl₃) δ 1.22 (t J= 7.89, 3H), 2.96 (q J = 7.89, 2H), 7.6 (bs, 2H), 7.8 (s, 1H).

### Example 15

### Preparation of N-3-bromobenzaldehyde oxime-4-O-Methylhydroxylamine)phenoxymethyl-copoly(styrene-1 %-divinylbenzene)-resin.

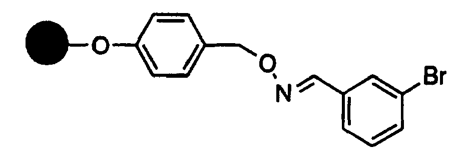

4-O-Methylhydroxylamine)phenoxymethyl-copoly(styrene-1%-divinylbenzene)-resin (105 mg; 0.08 mmol) is swelled in dichloromethane (DCM)(@ mL) for 10 minutes. Trimethylorthoformate (1 mL) and 3-bromo-benzaldehyde (500 mg; 2.7 mmol; 34 equiv.) is added to the resin and the mixture is shaken overnight. The slurry is then filtered, rinsed with dichloromethane (5 mL), DMF (5 mL x 3), H₂O (5 mL x 4), THF (5 mL x 10, and Et₂O (5 mL x 10). The resin is dried *in vacuo* at 40 °C for 12 hours. IR oxime stretch 1602 cm⁻¹. Elemental Analysis: calcd: Br, 5.52%; N, 1.04%. Found: Br, 5.76%; N, 1.08%.

### Example 16

### Preparation of N-3-(4-methoxphenyl)propan-1-oyl-4-O-methylhydroxylamine)phenoxymethyl-copoly(styrene-1 %-divinylbenzene)-resin.

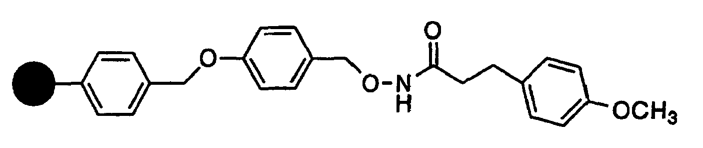

4-O-methylhydroxylamine)phenoxymethyl-copoly(styrene-1 %-divinylbenzene)-resin (1 g, 0.73 mmol) is allowed to swell in DMF for 10 minutes and then is treated with 3-(4-methoxyphenyl)propionic acid (0.658 g, 3.65 mmol) and DIC (0.46 g, 3.65 mmol). The mixture is shaken for 24 hours, then filtered and the residue is washed with DMF, DMF/H₂O, DMF, THF and ET₂O, and dried under vacuum at 40 °C to give N-3-(4-methoxphenyl)propan-1-oyl-4-O-methylhydroxylamine)phenoxymethyl-copoly(styrene-1%-divinylbenzene)-resin. IR: C=O 1698 cm⁻¹. Elemental Analysis: calcd: N, 1.02%. Found: N, 1.21 %.

### Example 17

### Preparation of N-hydroxy-3-(4-methoxyphenyl)propionamide.

N-hydroxy-3-(4-methoxyphenyl)propionamide is prepared by reaction of N-3-(4-methoxphenyl)propan-1-oyl-4-O-methylhydroxylamine)phenoxymethyl-copoly(styrene-1 %-divinylbenzene)-resin with TFA using the procedure of Example 7. ¹H NMR 300MHz (CDCl₃/. CD₃OD) δ 2.25 (t, 2H), 2.78 (t, 2H), 3.68 (s, 3H), 6.72 (d, 2H), 7.04 (d, 2H).

### Example 18

### Preparation of N-2-(4-bromophenyl)ethan-1-oyl-4-O-methylhydroxylamine)phenoxymethyl-copoly(styrene-1 %-divinylbenzene)-resin.

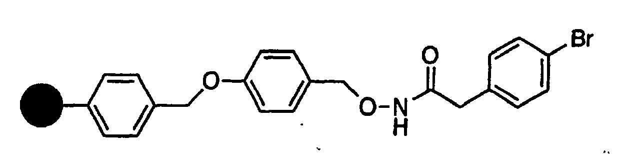

The title resin is prepared using the method of Example 16, except substituting 4-bromophenylacetic acid for 3-(4-methoxyphenyl)propionic acid. IR C=O 1713.9 cm⁻¹. Elemental analysis: calcd: Br, 5.8%; N, 1.02%. Found: Br, 8.29%, 8.18%; N, 0.97%, 0.96%.
EDS: Net X-ray Counts

| | K line | L line | M line |
|---|---|---|---|
| O: | 969 | 1024 | |
| C: | 2662 | 3003 | |
| Br: | | | 12855 10436 |

### Example 19

### Preparation of N-4-bromocinnamoyl-4-O-methylhydroxylamine)phenoxymethyl-copoly(styrene-1%-divinylbenzene)-resin.

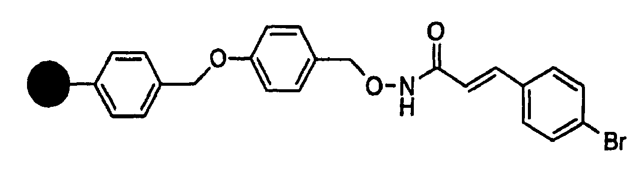

The title resin is prepared using the method of Example 16, except substituting 4-bromocinnamic acid for 3-(4-methoxyphenyl)propionic acid. IR:C=0 1671.7 cm⁻¹ (broad). Elemental analysis: Calcd: Br, 5.8%; N, 1.02%. Found: Br, 4.45%, 4.54%.
EDS: Net X-ray Count

| | K line | L line | M line |
|---|---|---|---|
| O: | 818 | 1365 | |
| C: | 4549 | 5059 | |
| Br: | | | 6384 5271 |

### Example 20

### Preparation of N-hydroxy-4-bromocinnamamide.

N-hydroxy-4-bromocinnamamide is prepared by treating N-4-bromocinnamoyl-4-O-methylhydroxylamine)phenoxymethyl-copoly(styrene-1%-divinylbenzene)-resin with TFA using the procedure of Example 7. LC MS (H-ISP) m/z 241/243 Br [M⁺], Area = 84%; ¹H NMR (300 Mhz, CDCl₃,CD₃OD) δ: 3.23 (s, 1H), 6.35 (d, J= 15.8), 7.3 (d, J= 7.9), 7.4 (d, J= 7.9), 7.6 (d, J= 15.8).

### Example 21

### Preparation of N-(4-chlorobenzoyl)-4-O-methylhydroxylamine)phenoxymethyl-copoly(styrene-1%-divinylbenzene)-resin.

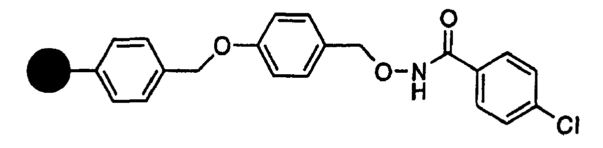

The title resin is prepared using the method of Example 16, except substituting 4-chlorobenzoic acid for 3-(4-methoxyphenyl)propionic acid. IR: C=O 1678 cm⁻¹. Elemental Analysis: calcd: Cl, 2.66%; N, 1.05%. Found: Cl, 2.39%; N, 1.02%.

### Example 22

### Preparation of N-Hydroxy-4-chlorobenzamide.

N-Hydroxy-4-chlorobenzamide is prepared by treating N-(4-chlorobenzoyl)-4-O-methylhydroxylamine)phenoxymethyl-copoly(styrene-1%-divinylbenzene)-resin with TFA using the procedure of Example 7. LC MS (H-ISP) m/z 172,174 (Cl) [M+H]⁺, Area = 96%; ¹H NMR (300 Mhz, DMSO-d₆) δ 7.48 (d*J* = 9.42, 2H), 7.69 (d *J* = 9.42, 2H), 8.9-9.2 (broad, 1H),11.28 (s, 1H).

### Example 23

### Peparation of N-methyl-N-(4-chlorobenzoyl)-4-O-methylhydroxylamine)phenoxymethyl-copoly(styrene-1 %-divinylbenzene)-resin.

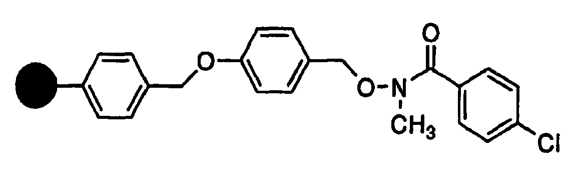

N-(4-chlorobenzoyl)-4-O-methylhydroxylamine)phenoxymethyl-copoly(styrene-1 %-divinylbenzene)-resin (0.1 g, 0.075 mmol, 0.75 mmol/g) is suspended in toluene (2 mL) and cooled to 5°C. The mixture is treated with methyl iodide (1.5 mmol, 0.21 g, 93µl) followed by DBU (0.22 mL, 0.228 g, 1.5 mmol). The reaction mixture is placed in a vortexer and allowed to warm to ambient temperature. Within a few minutes a copious white precipitate forms and the mixture is diluted further with toluene (2 mL). Agitation of the reaction mixture is continued for 18 hours. The N-methyl-N-(4-chlorobenzoyl)-4-O-methylhydroxylamine)phenoxymethyl-copoly(styrene-1%-divinylbenzene)-resin is filtered and washed with DMF, DMF/H₂O, DMF, THF, Et₂O and dried in *vacuo* at 40°C.

### Example 24

### Preparation of 1-(4-chlorophenyl)propan-1-one.

N-methyl-N44-chlorobenzoyl)-4-0-methylhydroxylan3ine)phenoxymethyl-copoly(styrene-l%-divinylbenzene)-resin is suspended in diethyl ether (0.7 mL) and treated with ethyl magnesium bromide (1.0 M in THF, 0.225 mL, 0.225 mmol). The reaction mixture is agitated for 18 hours on a wrist shaker and then quenched by the addition of 5% HCl in ethanol. Agitation is maintained for a further 30 minutes and the mixture is then filtered through a small plug of silica to remove the inorganic material. The filtrate is concentrated to afford 1-(4-chlorophenyl)propan-1-one. MS (EI-LRP) m/z 168/170 Cl [M⁺], 169/171 Cl [M+H]⁺;¹H NMR (300Mhz, CDCl₃) δ1.22 (t, 3H), 2.98 (q. 2H), 7.42 (d, 2H), 7.9 (d, 2H).

### Example 25

### Preparation of N-[3-((4-methoxyphenyl)sulfonyl)propan-1-oylcarbonyl]- 4-O-methylhydroxylamine)phenoxymethyl-copoly(styrene-1 %-divinylbenzene)-resin.

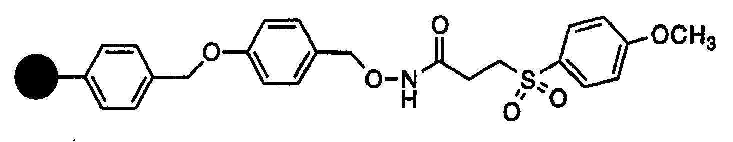

N-[3-((4-methoxyphenyl)sulfonyl)propan-1-oylcarbonyl]- 4-O-methylhydroxylamine)phenoxymethyl-copoly(styrene-1%-divinylbenzene)-resin is prepared using the method of Example 16, except substituting added 3-(4-methoxyphenylsulfonyl)propionic acid for 3-(4-methoxyphenyl)propionic acid. IR C=O 1691.6 cm⁻¹ (broad). Elemental Analysis: calcd: N, 1.02%; S, 2.34%. Found: N, 1.03%; S, 2.5%.

### Example 26

### Preparation of N-hydroxy-3-(4-metboxyphenylsulfonyl)propionamide.

N-hydroxy-3-(4-methoxyphenylsulfonyl)propionamide is prepared by treating N-[3-((4-methoxyphenyl)sulfonyl)propan-1-oylcarbonyl]- 4-0-methylhydroxylamine)phenoxymethyl-copoly(styrene-1 %-divinylbenzene)-resin with TFA using the procedure of Example 7. ¹H NMR (300Mhz, DMSO-d₆) δ 2.25 (t, 2H), 3.42 (t, 2H) 3.85 (s, 3H), 7.13 (d, 2H), 7.79 (d, 2H); LC MS (Ion Spray) m/z 259 [M⁺], Area = 44%

### Example 27

### Preparation of N-allyoxycarbonyl-4-(O-methylhydroxylamine)phenoxymethyl-copoly(styrene- % divinylbenzene) resin.

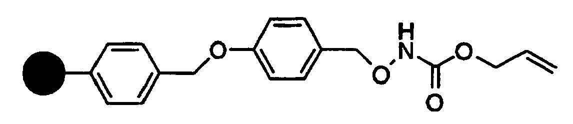

4-(O-methylhydroxylamine)phenoxymethyl-copoly(styrene-1% divinylbenzene) resin (2 g, 2 mmoles) is suspended in 15 ml of dichloromethane and shaken on a wrist shaker for 10 minutes and 284 mg (383 ul, 2.2 mmoles) of diisopropylethyl amine is added. The mixture is shaken for 30 minutes. Allyl chloroformate (265 mg, 233ul, 2.2 mmoles) is added and the mixture is shaken overnight. The N-allyoxycarbonyl-4-(O-methylhydroxylamine)phenoxymethyl-copoly(styrene-1% divinylbenzene) resin is washed with 15 ml of dichloromethane, THF (3x) and dichloromethane (3x) and dried *in vacuo .*

### Example 28

### Preparation of N-4-bromobenzyl-N-allyoxycarbonyl-4-(O-methylhydroxylamine)phenoxymethyl-copoly(styrene-1% divinylbenzene) resin.

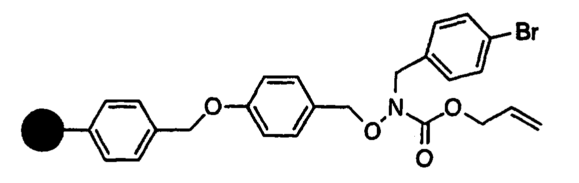

The N-allyoxycarbonyl-4-(O-methylhydroxylamine)phenoxymethyl-copoly(styrene-1% divinylbenzene) resin prepared in Example 27 is suspended in 15 mL of toluene. DBU (1,522 g,1.5 ml, 10 mmoles) and 4-bromobenzyl bromide (2.5 g,10 mmoles) are added and the mixture is shaken for 70 hours. The N-4-bromobenzyl-N-allyoxycarbonyl-4-(O-methylhydroxylamine)phenoxymethyl-copoly(styrene-1% divinylbenzene) resin is washed with 15 mL of DMF (3x), THF (3x) and dichloromethane (3x) and dried *in vacuo .*

### Example 29

### Preparation of N-4-bromobenzyl-4-(O-methylhydroxylamine)phenoxymethyl-copoly(styrene-1% divinylbenzene) resin.

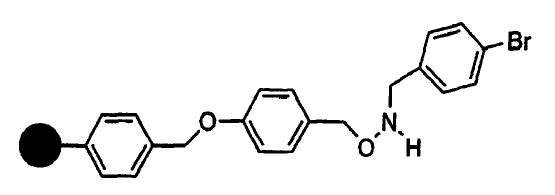

N-4-bromobenzyl,N-allyoxycarbonyl-4-(O-methylhydroxylamine)phenoxymethyl-copoly(styrene-1% divinylbenzene) resin, prepared as in Example 28, is swelled in 6 mL of THF. 6 mL of DMSO, 3 mL of 0.5 n Hcl. Pd(Ph₃P)₄ (347 mg, 15 weight %) is added and the mixture is shaken for 5 minutes. Morpholine (4.3 mL) is added and the mixture is shaken overnight. The reagents are drained off and the N-4-bromobenzyl-4-(O-methylhydroxylamine)phenoxymethyl-copoly(styrene-1 % divinylbenzene) resin is washed with DMF (2x), THF (2x), dichloromethane (2x), 0.5 % diisopropylethyl amine in dichloromethane (3x), 0.5 % sodium diethyldithiocarbamate in DMF (3x), DMF (3x), THF (3x) and dichloromethane (3x) and dried *in vacuo* overnight.

### Example 30

### Preparation of N-(indol-2-ylcarbonyl)-N-4-bromobenzyl-4-(O-methylhydroxylamine)phenoxymethyl-copoly(styrene-1 % divinylbenzene) resin.

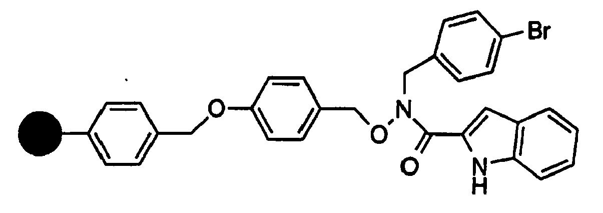

N-4-bromobenzyl-4-(O-methylhydroxylamine)phenoxymethyl-copoly(styrene-1% divinylbenzene) resin (1.17 g, 1 mmole), prepared as in Example 29, is suspended in 15 ml of DMF. Indole-2-carboxylic acid (483 mg, 3 mmoles) and 575.1 mg (3 mmoles) of 1(3-dimethylaminopropyl)-3-diethylcarbodiimide hydrochloride are added and the mixture is shaken for 16 hours. The N-(indol-2-ylcarbonyl)-N-4-bromobenzyl-4-(O-methylhydroxylamine)phenoxymethyl-copoly(styrene-1% divinylbenzene) resin is drained and washed with 15 ml of DMF (3x), THF/20% H₂O (3x), THF (3x), and dichloromethane (3x) and dried *in vacuo.*

### Example 31

### Preparation of Indole-2-carboxaldehyde.

Dry N-(indol-2-ylcarbonyl)-N-4-bromobenzyl-4-(O-methylhydroxylamine)phenoxymethyl-copoly(styrene-1% divinylbenzene) resin is swelled in 12 mL of THF, shaken and cooled at O°C for 30 minutes. LiAlH₄ (0.62 ml, 3 eq) is added and the mixture is shaken at O°C for 30 minutes. Saturated KHSO, solution (0.5 mL) and 0.3 mL of potassium, sodium tartrate solution are added and the mixture is shaken for 30 minutes while warming to room temperature. Excess H₂O is dried by adding dry Na₂SO₄, and shaking for 15 minutes more. The mixture is filtered under low nitrogen pressure and washed 3 more times with 8 mL of dichloromethane followed by filtration. The filtrate is dried with Na₂SO₄ and filtered twice through a short bed (1 inch) of silica gel 60 for column chromatography (particle size 0.040-0.063 mm) and the solvent is removed *in vacuo* to give indole-2-carboxaldehyde. ¹H NMR (CDCl₃) δ 9.84 (1H,s), 9.22 (1H,brs), 7.75 (1H,d), 7.14-7.48 (4H,m); MS (EI): m/z = 146 [M+H⁺].

### Example 32

### Preparation of N-(terr-butoxycarbonyl)phenylalanine aldehyde.

The desired compound is prepared according to the method of Examples 30 and 31, except substituting N*-(tert-*butoxycarbonyl)phenylalanine for indole-2-carboxaldehyde.

### Example 33

### Preparation of N-(3,4-dimethoxycinnamoyl)-4-O-methylhydroxylamine)phenoxymethyl-copoly(styrene-1%-divinylbenzene)-resin.

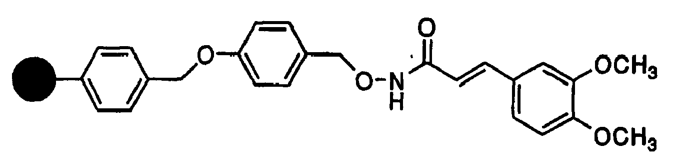

4-O-Methylhydroxylamine)phenoxymethyl-copoly(styene-1 %-divinylbenzene)-resin resin (1g, 1mmole) is washed with DMF (15 mL), then suspended in 15 mL of DMF and 624.6 mg (3 mmole, 3 x excess) of 3,4-dimethoxy cinnamic acid and 575.1 mg (3 mmoles, 3 x excess) of 1-(3-dimethylaminopropyl)-3-ethylcarbodiimide hydrochloride are added and the mixture is shaken for 16 hours. The resin is drained and washed with 15 mL of DMF (1x), THF/20% H₂O (3x), THF (3x), dichloromethane (3x) and dried under vacuum overnight.

### Example 34

### Preparation of N-4-bromobenzyl-N-(3,4-dimethoxycinnamoyl)-4-O-methylhydroxylamine)phenoxymethyl-copoly(styrene-1%-divinylbenzene)-resin.

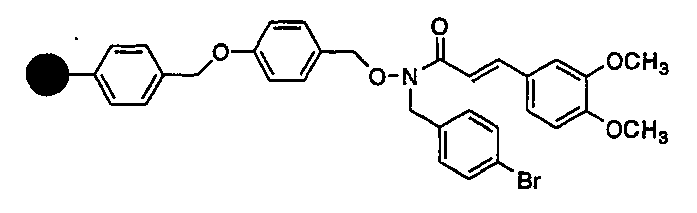

Dry N-(3,4-dimethoxycinnamoyl)-4-O-methylhydroxylamine)phenoxymethyl-copoly(styrene-1%-divinylbenzene)-resin is shaken in 15 mL of toluene for 10 minutes, then 0.9 mL (6 mmoles, 6 x excess) of DBU is added and the mixture is shaken for 2 hours p-Bromo benzyl bromide (1.5 g, 6 mmoles, 6 x excess) is added and the mixture is shaken for 3 days. The resin is dried overnight in *vacuo.*

### Example 35

### Preparation of 3-4-dimethoxycinnamaldehyde.

Dry N-4-bromobenzyl-N-(3,4-dimethoxycinnamoyl)-4-O-methylhydroxylamine)phenoxymethyl-copoly(styrene-1%-divinylbenzene)-resin is swelled in 12 mL of dry THF, shaken and cooled to 0°C for 30 minutes. LiAlH₄ in THF (0.5 mL, 2 equivalents) is added and the mixture is shaken at 0 °C for 30 minutes. Saturated aqueous KHSO₄ solution (0.5 mL) and potassium, sodium tartrate solution (0.3 mL) are added and the mixture is shaken for 30 minutes while warming to ambient temperature. Excess H₂O was dried by adding dry Na₂SO₄ and shaking for 15 minutes. The mixture is filtered under low nitrogen pressure, washed 3 times with 8 mL of dichloromethane and filtered. The filtrate is further dried with Na₂SO₄ and filtered through a short (1 inch) bed of silica gel 60 for column chromatography (particle size 0.040-0.063 mm) and the solvent is removed *in vacuo* to give 3,4-dimethoxycinnamaldehyde. ¹H NMR -(CDCl₃) δ 9.65 (1H,d), 7.40 (1H,d), 7.12 (1H,d), 7.06 (1H,s), 6.87 (1H,d), 6.60 (1H,dd), 3.90 (6H,s); MS (EI): m/z =193 [M+H⁺].

### Examples 36-43

The compounds of Examples 36-43 are prepared from the desired carboxylic acid starting material using the procedures of Examples 33-35.

### Example 36

Preparation of anthranilic aldehyde.

¹H NMR (CDCl₃) δ 9.88 (1H,s), 7,52-7.58 (1H,d), 7.11-7.38 (7H,m), 6.81 (1H,t); MS (EI): m/z = 198 [M+H⁺].

### Example 37

Preparation of 2-bibenzylic aldehyde.

¹H NMR (CDCl₃) δ10.18 (1H,s), 7.83 (1H,d), 7.14-7.52 (8H,m), 3.30 (2H,t), 2.87 (2H,t); MS (EI): m/z = 211 [M+H).

### Example 38

Preparation of 4-methoxy-2-quinoline aldehyde.

¹H NMR (CDCl₃) 810.17 (1H,s), 8.27 (1H,d), 8.18 (1H,d), 7.78 (1H,t), 7.62 (1H,t), 7.38 (1H,s), 4.12 (3H.s); MS (EI): m/z =188 [M+H⁺]

### Example 39

### Preparation of 3-acetamido benzaldehyde.

¹H NMR (CDCl₃) δ 9.98 (1H,s), 7.97 (1H,s), 7.86 (1H,d), 7.62 (1H,d), 7.48 (1H,t), 2.21 (3H,s). MS (EI): m/z = 164 [M+H⁺].

### Example 40

### Preparation of 4-(4-N-propylphenyl) benzaldehyde.

¹H NMR (CDCl₃) δ 10.02 (1H,s), 7,92 (2H,d), 7.72 (2H,d), 7.53 (2H,d), 7.26 (2H,d), 2.65 (2H,t), 1.68 (2H, dt), 0.95 (3H,t); MS (EI): m/z = 225 [M+H⁺].

### Example 41

### Preparation of 3-quinoline aldehyde.

¹H NMR (CDCl₃) 810.26 (1H,s), 9.38 (1H,s), 8.64 (1H,s), 8.20 (1H,d), 7.98 (1H,t), 7.89 (1H,t), 7.65 (1H,t); MS (EI); m/z =158 [M+H⁺].

### Example 42

### Preparation of 3-(3,4-methylenedioxy) propionaldehyde.

¹HNMR (CDCl₃) δ 9.80 (1H,s), 7.60-7.74 (3H,m), 5.92 (2H,s), 2.88 (2H,t), 2,74 (2H,t);MS (EI): m/z =179 [M+H⁺].

### Example 43

### Preparation of 2-phenyl-4-qninoline aldehyde.

¹H NMR (CDCl₃) δ 10.58 (1H,s), 9.00 (1H,d) 8.19-8.30 (4H,m), 7.82 (1H,t), 7.70 (1H,t), 7.47-7.59 (3H,m); MS (EI): m/z = 234 [M+H⁺].

### Example 44

Preparation of 4-carboxy-2,3,5,6-tetrafluorophenoxymethyl-copoly(styrene-1% divinylbenzene) resin.

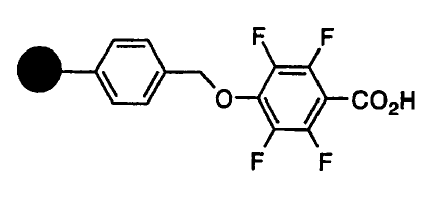

Merrifield resin (2 mmol/g, 600 mg, 1.2 mmol) is swelled in anhydrous DMF (20 mL).. 2,3,5,6-tetrafluoro-4-hydroxy benzoic acid hydrate (2.28 g, 10 mmol) and cesium carbonate (3.26 g, 10 mmol) are added and the reaction mixture is heated at 85°C for 12 hours with gentle agitation. The reaction mixture is filtered and the 4-carboxy-2,3,5,6-tetrafluorophenoxymethyl-copoly(styrene-1% divinylbenzene) resin is washed with DMF (5x), 20% aqueous DMF (5x), THF (5x) and dichloromethane and dried overnight in vacuo. IR (microscope, cm-1): 1640 (C=O); ¹⁹F NMR (nanoprobe) -144.4 ppm, -160.2 ppm.

### EDS

The energy dispersive x-ray measurements are made with an Electroscan Scanning Electron Microscope with an attached PGT digital detector. The beads are mounted on aluminum stubs and tested without a conductive coating. The net x-ray counts are reported after correction for the background. No corrections are made for atomic number, fluorescence or absorption.

## Claims

1. A polymeric tetrafluorophenyl hydroxylamine resin compound of formula 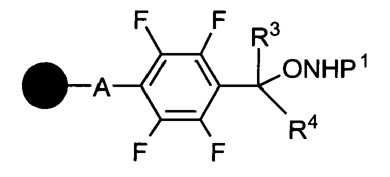 wherein
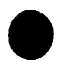 is a solid support;
A is absent or a group of formula -X¹-Z- wherein
X¹ is -CHR- or -CHR-Y-CO-(CH₂)ₙ- wherein R is H, alkyl, phenyl, or phenyl substituted with -H, alkyl, alkoxy, halogen, nitrile or -NO₂,
Y is -O- or -NH-,
n is an integer from 1 to 6, and
Z is -O- or -NH-;
R³ and R⁴ are independently -H, alkyl, phenyl, or phenyl substituted with one or more substituents selected from alkyl, alkoxy, halogen, nitrile and -NO₂; and
P¹ is H or an amine protecting group.

2. A polymeric tetrafluorophenyl hydroxylamine resin compound according to claim 1 wherein A is -O-.

3. A polymeric tetrafluorophenyl hydroxylamine resin compound according to claim 2 wherein one of R³ and R⁴ is H and the other is H or 2,4-dimethoxyphenyl.

4. A polymeric tetrafluorophenyl hydroxylamine resin compound according to claim 3 wherein P¹ is H.

5. A polymeric hydroxylamine resin compound according to claim 3 wherein P¹ is allyloxycarbonyl.

6. A polymeric hydroxylamine resin compound according to claim 3 which is 4-(O-methylhydroxylamine)-2,3,5,6-tetrafluorophenoxymethyl-copoly(styrene-1% divinylbenzene) resin, or 4-(2',4'-dimethoxyphenyl-O-methylhydroxylamine)-2,3,5,6-tetrafluorophenoxymethyl-copoly(styrene-1% divinylbenzene) resin.

7. A polymeric N-protected hydroxylamine resin compound of formula 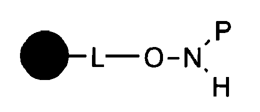 wherein
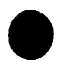 is a solid support;
L is absent or a linking group;and
P is an amine protecting group, provided that P is other than 4-methoxybenzyl or 2,4-dimethoxybenzyl.

8. A polymeric N-protected hydroxylamine resin compound according to claim 7 wherein L is a linking group.

9. A polymeric N-protected hydroxylamine resin compound according to claim 8 wherein L is a linking group of formula 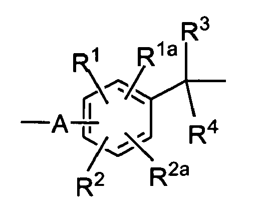 wherein
A is absent or a group of formula -X¹-Z- wherein
X¹ is -CHR- or -CHR-Y-CO-(CH₂)ₙ- wherein R is H, alkyl, phenyl, or phenyl substituted with -H, alkyl, alkoxy, halogen, nitrile or -NO₂,
Y is -O- or -NH-,
n is an integer from 1 to 6, and
Z is -O- or -NH-;
R¹, R^{1a}, R², and R^{2a} are independently -H, alkyl, alkoxy, halogen, nitrile or -NO₂; and
R³ and R⁴ are independently -H, alkyl, phenyl, or phenyl substituted with one or more substituents selected from alkyl, alkoxy, halogen nitrile and -NO₂;
or one of R¹ and R² taken together with one of R³ and R⁴ and the carbon atoms to which they are attached defme a linking group of formula 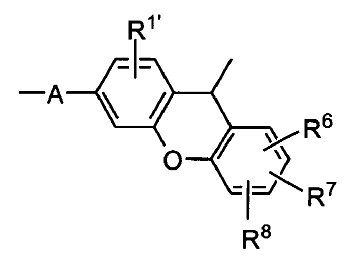 wherein
R^{1'} is -H, alkyl, alkoxy, halogen, nitrile or -NO₂; and
R⁶, R⁷ and R⁸ are independently selected from -H, alkyl, alkoxy, halogen, nitrile or -NO₂.

10. A polymeric N-protected hydroxylamine resin compound according to claim 9 wherein P is allyloxycarbonyl, benzyloxycarbonyl, p-methoxybenzyloxycarbonyl, p-nitrobenzyloxycarbonyl, trimethylsilylethoxycarbonyl, 2,4-dimethoxybenzyloxycarbonyl, o-nitrobenzyloxycarbonyl, o-nitrobenzylsulfonyl, p-nitrobenzylsulfonyl, and 2-nitro-4-trifluoromethylbenzenesulfonyl.

11. A polymeric N-protected hydroxylamine resin compound according to claim 10 wherein P is allyloxycarbonyl.

12. A polymeric N-protected hydroxylamine resin compound according to claim 11 which is N-allyloxycarbonyl-4-(O-methylhydroxylamine)phenoxymethyl-copoly(styrene-1% divinylbenzene) resin, N-allyloxycarbonyl-4-[4-(O-methylhydroxylamine)-3-methoxyphenoxy]-(N-4-methylbenzhydryl)-butyramide-copoly(styrene-1 %-divinylbenzene)-resin, N-allyloxycarbonyl-4-(2',4'-dimethoxyphenyl-O-methylhydroxylamine)-phenoxymethyl-copoly(styrene-1 % divinylbenzene) resin, N-allyloxycarbonyl-4-[4-(1-aminoxyethyl)-2-methoxy-5-nitrophenoxy]-(N-4-methylbenzhydryl)-butyramide-copoly(styrene-1% divinylbenzene) resin, N-allyloxycarbonyl-O-hydroxylamine-21-chlorotrityl-copolystyrene- %-divinylbenzene-resin, N-allyloxycarbonyl-O-hydroxylamine-trityl-copolystyrene-1 %-divinylbenzene-resin, N-allyloxycarbonyl-5-(4-O-methylhydroxylamine -3,5-dimethoxyphenoxy)-valeric acid-copolystyrene-1%-divinyl benzene resin, N-allyloxycarbonyl-4-(O-methylhydroxylamine)-2,3,5,6-tetrafluorophenoxymethyl-copoly(styrene-1% divinylbenzene) resin, N-allyloxycarbonyl-4-(2',4'-dimethoxyphenyl-O-methylhydroxylamine)-2,3,5,6-tetrafluorophenoxymethyl-copoly(styrene-1% divinylbenzene) resin, N-allyloxycarbonyl-4-O-methylhydroxylamine-3-methoxyphenoxy-copolystyrene-1 %-divinyl benzene resin, or N-allyloxycarbonyl-3-hydroxy-xanthydrolamine-copolystryene-1%-divinylbenzene resin.

13. A polymeric N-protected hydroxylamine resin compound according to claim 11 which is N-allyloxycarbonyl-4-(O-methylhydroxylamine)phenoxymethyl-copoly(styrene-1% divinylbenzene) resin.

14. A process for preparing a hydroxamic acid compound of formula 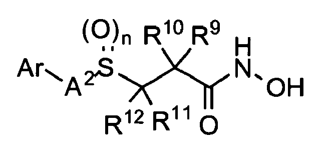 wherein
A² is a direct bond or an alkylene, or NR¹³ wherein R¹³ is hydrogen or alkyl;
R⁹ is a group of formula -L¹-R¹⁴, where L¹ represents a direct bond or a straight- or branched C₁₋₆alkylene chain optionally substituted by alkoxy, aryl, carboxy, cyano, cycloalkyl, halogen, heteroaryl, hydroxyl, or oxo, and R¹⁴ is hydrogen, aryl, carboxy, cyano, cycloalkyl, cycloalkenyl, cyclocarbamoyl, cycloimidyl, heterocycloalkyl, heteroaryl, -NH-C(=O)-NH₂, (N-carbamoyl)cyclic amine, -C=N-O-C(=O)-NH₂, -C(=O)-NY¹Y² [where Y' and Y² are independently hydrogen, alkyl, arylalkyl, and aryl, or the substituent Y¹Y²N-forms a 4-6 membered cyclic amine which optionally contains an additional heteroatom selected from O, S, NH or NR¹³], -NY¹SO₂aryl, -NHR¹³, -SR¹³ or -OR¹³ ; or a group L²-R¹⁵ where L² represents a straight- or branched-carbon chain comprising from 2 to about 6 carbon atoms, optionally substituted with carboxy or cyano, which contains a double or triple carbon-carbon bond, or is interrupted by an oxygen or sulfur atom, a phenylene, imino (-NH-) or alkylimino linkage, or a sulfmyl or sulfonyl group, and R¹⁵ is hydrogen, aryl, carboxy, cyano, cycloalkyl, cycloalkenyl, heterocycloalkyl or heteroaryl; or R⁹ and R¹⁰ taken together with the atom to which they are attached form a ring; or R⁹ and R¹¹ taken together with the atoms through which they are attached form a ring;
R¹⁰ and R¹² are independently hydrogen or alkyl; or R¹⁰ and R¹² together form a bond;
R¹¹ represents a group -L³-R¹⁶ where L³ represents a direct bond or a straight- or branched C₁₋₆alkylene chain optionally substituted by by alkoxy, aryl, carboxy, cyano, cycloalkyl, halogen, heteroaryl, hydroxyl, or oxo; or L³ represents a straight- or branched-carbon chain comprising from 2 to about 6 carbon atoms which contains a double or triple carbon-carbon bond, or is interrupted by an oxygen or sulfur atom, a phenylene, imino (-NH-) or alkylimino linkage, or a sulfinyl or sulfonyl group; and R¹⁶ is hydrogen, aryl, cycloalkyl, cycloalkenyl, cyclocarbamoyl, cycloimidyl, heterocycloalkyl, heteroaryl, -NH-C(=O)-NH₂, (N-carbamoyl)cyclic amine, -C=N-O-C(=O)-NH₂, -C(=O)-NY¹Y² [where Y¹ and Y² are independently hydrogen, alkyl, arylalkyl, and aryl, or the substituent Y¹Y²N- forms a 4-6 membered cyclic amine which optionally contains an additional heteroatom selected from O, S, NH or NR¹³], -NY¹SO₂aryl, or R¹¹and R⁹ together with the atoms to which they are attached form a ring; or R¹¹ and R¹² together with the atom to which they are attached form a ring;
Ar is a group chosen from: 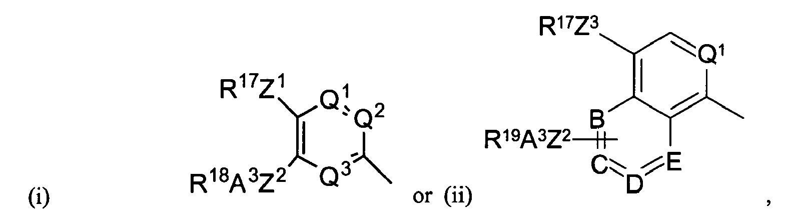 where R¹⁷ is a straight- or branched-chain alkyl group of 1 to about 6 carbon atoms, optionally substituted by one or more halogen atoms, or when Z³ is a direct bond R¹⁷ may also represent a hydrogen atom, alkenyl or alkynyl group;
R¹⁸ represents an optionally substituted cycloalkyl, cycloalkenyl, heterocycloalkyl, aryl, partially saturated bicycloaryl or heteroaryl group;
R¹⁹ represents R²⁰, -OR²⁰, -SR²⁰, -SOR²⁰ ,-SO₂R²⁰, -SO₂NR²⁰R²¹, -NR²⁰SO₂R²¹, -NR²⁰R²¹, -O(C=O)NR²⁰R²¹, -NR²⁰C(=O)R²¹, -N(OH)C(=O)R²⁰, or -C(=O)N(OH)R²¹, where R²⁰ and R²¹, which may be the same or different, each represent a hydrogen atom, or an alkyl, alkenyl, heterocycloalkyl, cycloalkyl, cycloalkenyl, aryl, heteroaryl, arylalkyl or heteroarylalkyl group, or the group NR²⁰R²¹ represents a 5 to 7 membered cyclic amine optionally containing one or more additional heteroatom selected from O, N, or S), wherein where R¹⁸ represents a substituted cycloalkyl group, the cycloalkyl group is substituted by one or more (e.g. 1, 2 or 3) substituents chosen from OR²³, SR²⁴, SOR²⁴, SO₂R²⁴, NH₂, NR¹³R²⁴, =NOR²⁴, =NOH, =NNHR²⁴ =NOCONHR²⁴ =NCO₂R²⁴ SOR²⁴, NHCOR²⁴, NHSO₂R²⁴, SO₂NR¹³R²⁴ R²³, CONHR²⁴ CONHCH₂CO₂R¹³ CONR²⁴R¹³ or N₃;
wherein R²³ is hydrogen, alkyl, cycloalkyl, aryl, arylalkyl, heteroaryl or heteroarylalkyl; R²⁴ is alkyl, cycloalkyl, cycloalkenyl, heterocycloalkyl, aryl, arylalkyl, heteroaryl or heteroarylalkyl; or the substituent NR¹³R²⁴ forms a 4-7 membered cyclic amine which optionally contains an additional heteroatom selected from O, S, NH or NR¹³, and wherein R¹⁸ represents a substituted heterocycloalkyl group containing a nitrogen atom the ring is substituted on one or more (e.g. 1, 2 or 3) of the ring carbon atoms and the substituents are chosen from oxo, cyano, CO₂R¹³, CONHCH₂CO₂R¹³, aryl, arylalkyl, alkyl or hydroxyalkyl, and/or is substituted on the ring nitrogen atom and the substituent is chosen from R¹³, (CH₂)nCO₂H, (CH₂)nCO₂R²⁴, (CH₂)nCONR¹³R²⁴, (CH₂)nCOR²⁴, CONH₂, CONHR²⁴, COR²⁴, SO₂R²⁴, or OR²⁴;
A³ represents a direct bond, a straight- or branched C₁₋₆alkylene chain optionally substituted by halogen, hydroxyl, alkoxy, oxo, cycloalkyl, aryl or heteroaryl; or A³ represents a straight- or branched-carbon chain comprising from 2 to about 6 carbon atoms which contains a double or triple carbon-carbon bond, or is interrupted by an oxygen or sulfur atom, a phenylene, imino (-NH-) or alkylimino linkage, or a sulfmyl or sulfonyl group,
Z¹ and Z³ each represents an oxygen or sulfur atom, a direct bond or NH;
Z² represents an oxygen or sulfur atom, or a direct bond;
B, C, D, and E independently represent a carbon or heteroatom selected from O, S, N, NOR²² or NR²² where R²² is hydrogen or a C₁₋₄straight- or branched-chain alkyl, aryl, arylC₁₋₄alkyl, heteroaryl or heteroarylC₁₋₄alkyl group, or three of B, C, D or E represent a carbon or heteroatom selected from O, N, NR²², or S and the other represents a direct bond; but excluding compounds where two O or S atoms are in adjacent positions, and the bonds joining B, C, D and E may be single or double bonds;
Q1, Q2 and Q3, which may be the same or different, each represents a CH or CX¹ linkage or a nitrogen atom; and X¹ represents a halogen atom; and
n is 0, 1 or 2; or
an N-oxide thereof, prodrug thereof, acid isostere thereof, pharmaceutically acceptable salt thereof, or solvate thereof,
comprising
(a) coupling a carboxylic acid compound of formula 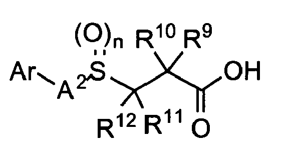 to a polymeric hydroxylamine resin compound of formula 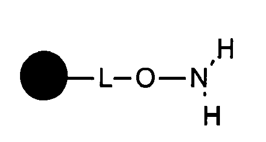 to form a polymeric hydroxamic resin compound of formula 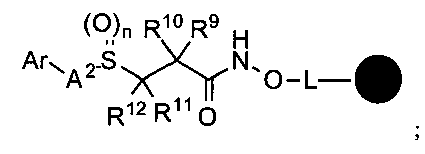 and
(b) treating the polymeric hydroxamic acid resin compound with acid.

15. The process of claim 14 wherein the acid is trifluoroacetic acid.

16. A process for the preparation of a polymeric oxime ether resin compound of formula 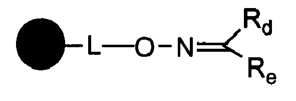 Wherein 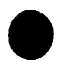 is a solid support, L is absent or a linking group and R_{d} and Rₑ are independently H, aliphatic or aromatic, comprising reacting a polymeric hydroxylamine resin compound of formula 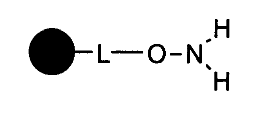 wherein 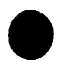 is a solid support and L is absent or a linking group, with a carbonyl compound of formula

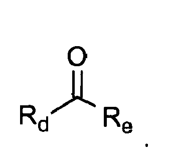

17. A process for the preparation of an α-amine compound of formula 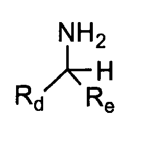 wherein R_{d} and Rₑ are independently H, aliphatic or aryl, provided that R_{d} and Rₑ are not both H, comprising reductively cleaving the α-amine compound from a polymeric oxime ether resin compound of formula 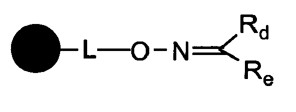 wherein 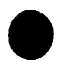 is a solid support and L is absent or a linking group.

18. The process of claim 17 wherein the reductive cleavage is accomplished using a hydride reducing agent.

19. The process of claim 18 wherein the reductive cleavage is accomplished by sequential treatment of the polymeric oxime ether resin compound with NaBH₃CN and LiA1H₄.

20. A process for the preparation of a substituted α-amine compound of formula 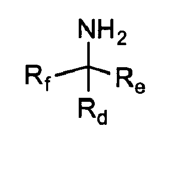 wherein R_{d} and Rₑ are independently H, aliphatic or aomatic, provided that R_{d} and Rₑ are not both H, and R_{f} is aliphatic or aromatic,
comprising
(a) reacting a polymeric oxime ether compound of formula 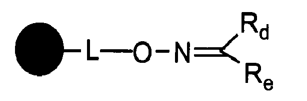 wherein 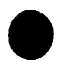 is a solid support, L is absent or a linking group, with an organometallic reagent of formula R_{f}M wherein R_{f} is an aliphatic or aromatic anion and M is a metal cation, to form a polymeric α-substituted hydroxylamine resin compound of formula 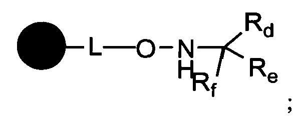 and
(b) reductively cleaving the α-substituted hydroxylamine resin compound.

21. The process of claim 20 wherein M is Li or MgX wherein X is Br or Cl.

22. The process of claim 21 wherein the reductive cleavage is accomplished using a hydride base.

23. The process of claim 22 wherein the hydride base is BH₃-THF or LiA1H₄.

24. A process for the preparation of a lactone compound of formula 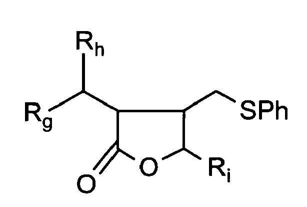 wherein Rg, Rₕ and R; are aliphatic or aromatic and Ph is phenyl,
comprising
(a) treating an α,β-unsaturated polymeric hydroxamic acid ester resin compound of formula 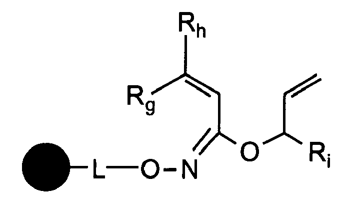 wherein 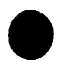 is a solid support, L is absent or a linking group, with thiophenol and a radical initiator to form a polymeric oximyl lactone compound of formula 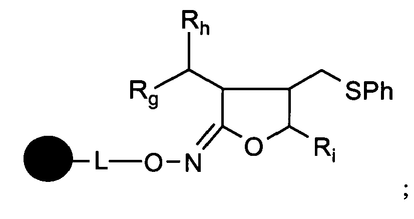 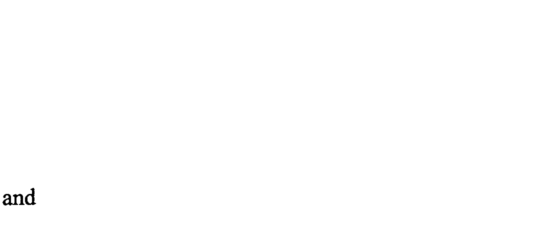
(b) treating the polymeric oximyl lactone compound with aqueous acid.

25. The process of claim 24 wherein the radical initiator is 2,2'-azobisisobutyronitrile.

26. The process of claim 25 wherein the aqueous acid is aqueous HCl.

27. The process of claim 24 wherein the α,β-unsaturated polymeric hydroxamic acid ester resin compound of formula 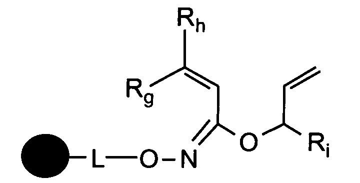 wherein 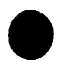 ,L and R_{g} and Rₕ and Rᵢ are defined therein is prepared by reacting a polymeric hydroxylamine resin compound of formula 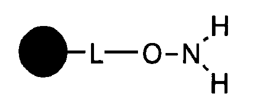 with an α,β-unsaturated carboxylic acid ester compound of formula

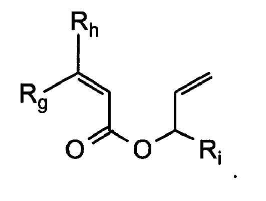

28. A process for the preparation of an α-cyclic hydroxylamine compound of formula 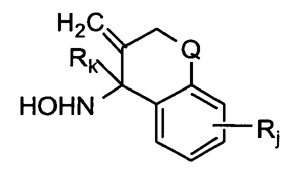 wherein Rⱼ and Rₖ are aliphatic or aromatic and Q is -O- or -CH₂-,
comprising
(a) treating a polymeric acetophenone oxime compound of formula 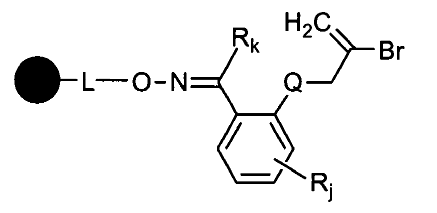 wherein 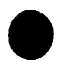 is a solid support and L is absent or a linking group,
with trialkyltin hydride and a radical initiator to form a polymeric α-cyclic hydroxylamine resin compound of formula 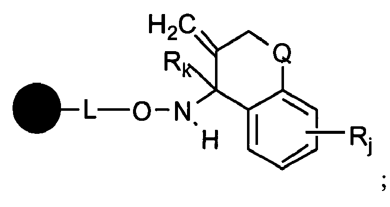 and
(b) treating the polymeric α-cyclic hydroxylamine resin compound with aqueous acid.

29. A process for the preparation of a α-cyclic amino compound of formula 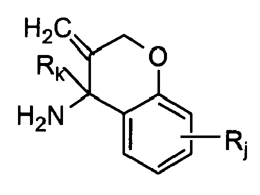 wherein Rⱼ and Rₖ are aliphatic or aromatic and Q is -O- or -CH₂-, comprising reductively cleaving a polymeric α-cyclic hydroxylamine resin compound of formula 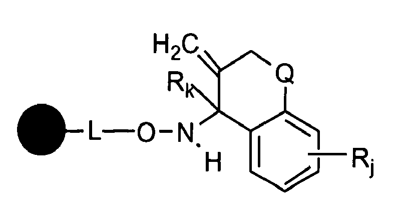 wherein 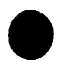 is a solid support and L is absent or a linking group.

30. A process for the preparation of a α-cyclic hydroxylamine compound of formula 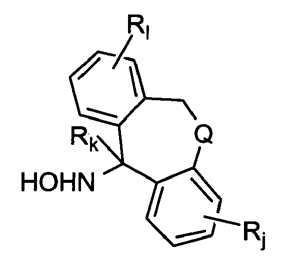 wherein Rⱼ, Rₖ and R₁ are aliphatic or aromatic and Q is -O- or -CH₂-,
comprising
(a) treating a polymeric acetophenone oxime compound of formula 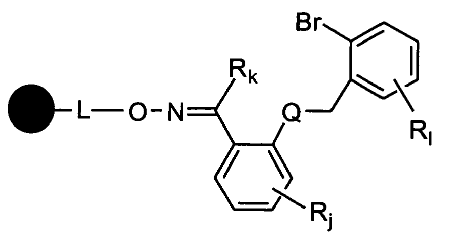 wherein 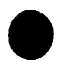 is a solid support and L is absent or a linking group,
with trialkyltin hydride and a radical initiator to form a polymeric α-cyclic hydroxylamine resin compound of formula 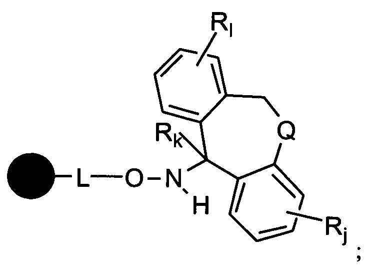 and
(b) treating the polymeric α-cyclic hydroxylamine resin compound with aqueous acid.

31. The process of claim 28 or 30 wherein the trialkyltin hydride is tri-n-butyltin hydride.

32. The process of claim 31 wherein the radical initiator is 2,2'-azobisisobutyronitrile.

33. A process for the preparation of a α-cyclic amino compound of formula 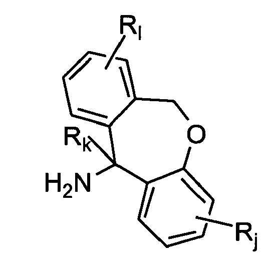 wherein Rⱼ, Rₖ and Rₗ are aliphatic or aromatic and Q is -O- or -CH₂-, comprising reductively cleaving a polymeric α-cyclic hydroxylamine resin compound of formula 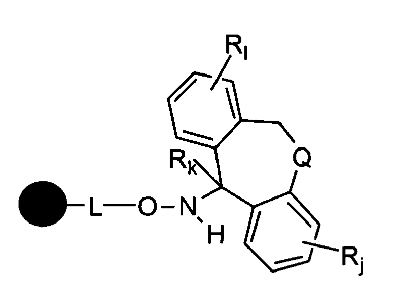 wherein 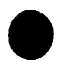 is a solid support and L is absent or a linking group.

34. The process of claim 29 or 33 wherein the reductive cleavage is accomplished using a hydride reducing agent.

35. The process of claim 34 wherein the hydride reducing agent is LiA1H₄.

36. The process of any one of claims 16, 17, 23, 27, 28, 30 or 35 wherein L is a linking group.

37. The process of claim 36 wherein L is a linking group of formula 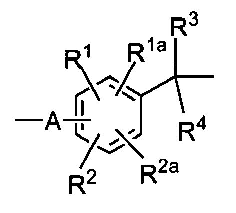 wherein
A is absent or a group of formula -X¹-Z- wherein
X¹ is -CHR- or -CHR-Y-CO-(CH₂)ₙ- wherein R is H, alkyl, phenyl, or phenyl substituted with -H, alkyl, alkoxy, halogen, nitrile or -NO₂,
Y is -O- or -NH-,
n is an integer from 1 to 6, and
Z is -O- or -NH-;
R¹, R^{1a}, R² and R^{2a} are independently -H, alkyl, alkoxy, halogen, nitrile or -NO₂; and
R³ and R⁴ are independently -H, alkyl, phenyl, or phenyl substituted with one or more substituents selected from alkyl, alkoxy, halogen, nitrile and -NO₂;
or one of R¹ and R² taken together with one of R³ and R⁴ and the carbon atoms to which they are attached define a linking group of formula 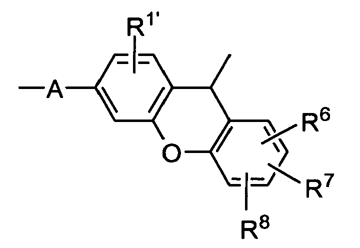 wherein
R^{1'} is -H, alkyl, alkoxy, halogen, nitrile or -NO₂; and
R⁶, k⁷ and R⁸ are independently selected from -H, alkyl, alkoxy, halogen, nitrile or -NO₂.
